(19) Europäisches Patentamt

European Patent Office

Office européen des brevets

(11) EP 0 628 633 B1

(12) EUROPEAN PATENT SPECIFICATION

(45) Date of publication and mention
of the grant of the patent:
**08.01.2003 Bulletin 2003/02**

(51) Int Cl.7: **C12N 15/38**, A61K 39/245
// C07K14/03

(21) Application number: **94202224.5**

(22) Date of filing: **04.04.1985**

(54) **Recombinant herpes simplex gB-gD vaccine**

Rekombinanter Herpes Simplex gB-gD-Impfstoff

Vaccin gB-gD récombinant contre l'herpès simplex

(84) Designated Contracting States:
**AT BE CH DE FR GB IT LI LU NL SE**

(30) Priority: **06.04.1984 US 597784**
**17.07.1984 US 631669**

(43) Date of publication of application:
**14.12.1994 Bulletin 1994/50**

(62) Document number(s) of the earlier application(s) in
accordance with Art. 76 EPC:
**85902226.1 / 0 175 787**

(73) Proprietor: **CHIRON CORPORATION**
**Emeryville California 94608-2916 (US)**

(72) Inventors:
• **Burke, Rae Lyn**
**San Francisco, CA 94122 (US)**
• **Pachl, Carol**
**Oakland, CA 94611 (US)**
• **Vanlenzuela, Pablo D.T.**
**San Francisco, CA 94117 (US)**
• **Urdea, Mickey S**
**San Francisco, CA 94122 (US)**

(74) Representative: **Hallybone, Huw George**
**Carpmaels and Ransford,**
**43 Bloomsbury Square**
**London WC1A 2RA (GB)**

(56) References cited:
**EP-A- 0 100 521**          **EP-A- 0 168 662**
**EP-A- 0 170 169**

• **SCIENCE vol. 218 , 22 October 1982 ,
LANCASTER, PA US pages 381 - 384
R.J.WATSON ET AL. 'HERPES SIMPLEX VIRUS
TYPE-1 GLYCOPROTEIN D GENE: NUCLEOTIDE
SEQUENCE AND EXPRESSION IN
ESCHERICHIA COLI.'**
• **NATURE. vol. 302, no. 5903 , 3 March 1983 ,
LONDON GB pages 72 - 74 J.H. WEIS ET AL. 'AN
IMMUNOLOGICALLY ACTIVE CHIMAERIC
PROTEIN CONTAINING HERPES SIMPLEX TYPE
1 GLYCOPROTEIN D.'**
• **IMMUNOLOGY vol. 49, no. 2 , June 1983 ,
OXFORD, GB pages 343 - 352 W.L. CHAN
'PROTECTIVE IMMUNIZATION OF MICE WITH
SPECIFIC HSV-1 GLYCOPROTEINS.'**
• **BIOTECHNOLOGY vol. 2, no. 6 , June 1984 , NEW
YORK US pages 527 - 532 L.A. LASKY ET AL.
'PROTECTION OF MICE FROM LETHAL HERPES
SIMPLEX VIRUS INFECTION BY VACCINATION
WITH A SECRETED FORM OF CLONED
GLYCOPROTEIN D.'**

**Description**

BACKGROUND OF THE INVENTION

Field of the Invention

The herpesviruses include the herpes simplex viruses, comprising two closely related variants designated types 1 (HSV-1) and 2 (HSV-2). These types cross react strongly but can be distinguished by neutralization titrations. HSV-1 and HSV-2 are responsible for a variety of human diseases, such as skin infections, genital herpes, viral encephalitis and the like.

The herpes simplex virus is a double stranded DNA virus having a genome of about 150 to 160kbp packaged within an icosahedral nucleocapsid enveloped in a membrane. The membrane includes a number of virus-specific glycoproteins, the most abundant of which are gB, gC, gD and gE, where gB and gD are cross-reactive between types 1 and 2.

It is a matter of great medical and scientific interest to provide safe and effective vaccines against both HSV-1 and HSV-2. One promising approach has been the use of isolated glycoproteins which have been shown to provide protection when injected into mice subsequently challenged with live virus. However, the availability of the Herpes Simplex glycoproteins has heretofore been primarily dependent upon the growth of the virus and the isolation of the membranous proteins. The problems of commercial production of the glycoproteins associated with the handling of a dangerous pathogen, the maintenance of the virus in cell culture, the isolation of the glycoproteins free of the viral genome or portions thereof, have substantially precluded the use of the glycoproteins as vaccines. It would therefore be desirable to provide vaccines employing glycoproteins produced by methods other than by growth of the virus and isolation of the membranous proteins.

Description of the Prior Art

Eberle and Mou, J. of Infectious Diseases (1983) 148:436-444 report the relative titers of antibodies to individual polypeptide antigens of HSV-1 in human sera. Marsden et al., J. of Virology (1978) 28:624-642 report the location of a gene for a 117 kilo-dalton (kd) glycoprotein to lie within 0.35-0.40 map units on the genetic map of HSV by intertypic recombination between HSV-1 and HSV-2. Ruyechan et al., ibid. (1979) 29:677-697 also report the mapping of glycoprotein B gene to lie between 0.30-0.42 map units. Skare and Summers, Virology (1977) 76:581-595, report endonuclease cleavage sites for EcoRI, XbaI and HindIII on HSV-1 DNA. Roizman, Ann. Rev. Genetics (1979) 13:25-57 reports the organization of the HSV genomes. DeLucca et al., Virology (1982) 122:411 map several phenotypic mutants thought to lie in the gB1 structural gene between 0.345 to 0.368 map units.

Subunit vaccines extracted from chick embryo cells infected with HSV-1 or HSV-2 are described in U.S. Patents Nos. 4,317,811 and 4,374,127. See also, Hilfenhaus et al., Develop. Biol. Standard (1982) 52:321-331, where the preparation of a subunit vaccine from a particular HSV-1 strain (BW3) is described. Roizman et al., ibid. (1982) 52:287-304, describe the preparation of non-virulent HSV-1 x HSV-2 recombinants and deletion mutants which are shown to be effective in immunizing mice. Watson et al., Science (1982) 218:381-384 describe the cloning and low level expression of the HSV-1 gD gene in E. coli, as well as expression of a cloned fragment by injection into the nuclei of frog oocytes. They also present the nucleotide sequence for the gD gene. Weis et al., Nature (1983) 302:72-74 report higher level expression of gD in E. coli. This polypeptide elicits neutralizing antibodies in rabbits. Berman et al., Science (1983) 222:524-527 report the expression of glycoprotein D in mammalian cell culture. Lasky et al., Biotechnology (June 1984) 527-532 report the use of this glycoprotein D for the immunization of mice. Cohen et al., J. Virol. (1984) 49:102-108 reports the localization and chemical synthesis of a particular antigenic determinant of gD, contained within residues 8-23 of the mature protein. Berman et al., Science (1985) 227:1490-1492 report the use of recombinant glycoprotein D for the immunization of guinea pigs.

SUMMARY OF THE INVENTION

Novel vaccines against Herpes Simplex Virus Types 1 and 2 and methods for their production are provided. These vaccines employ virus specific polypeptides produced by recombinant DNA technology. Particularly, HSV gD was produced in modified yeast hosts and employed as vaccines either separately or in combination.

BRIEF DESCRIPTION OF THE DRAWINGS

Fig. 1 is a flow diagram of the construction of plasmid pSV1/dhfr.

Fig. 2 is a flow diagram of the construction of gB vectors, plasmids pHS112 and pHS114.

Fig. 3 represents the construction of plasmids pYHS109 and pYHS110 which carry synthetic sequences gD-A and gD-B, respectively, as described in the Experimental section hereinafter.

Fig. 4 is a partial restriction map of the gD region which notes the location of all the gD sequences inserted into yeast expression vectors, as described in the Experimental section hereinafter.

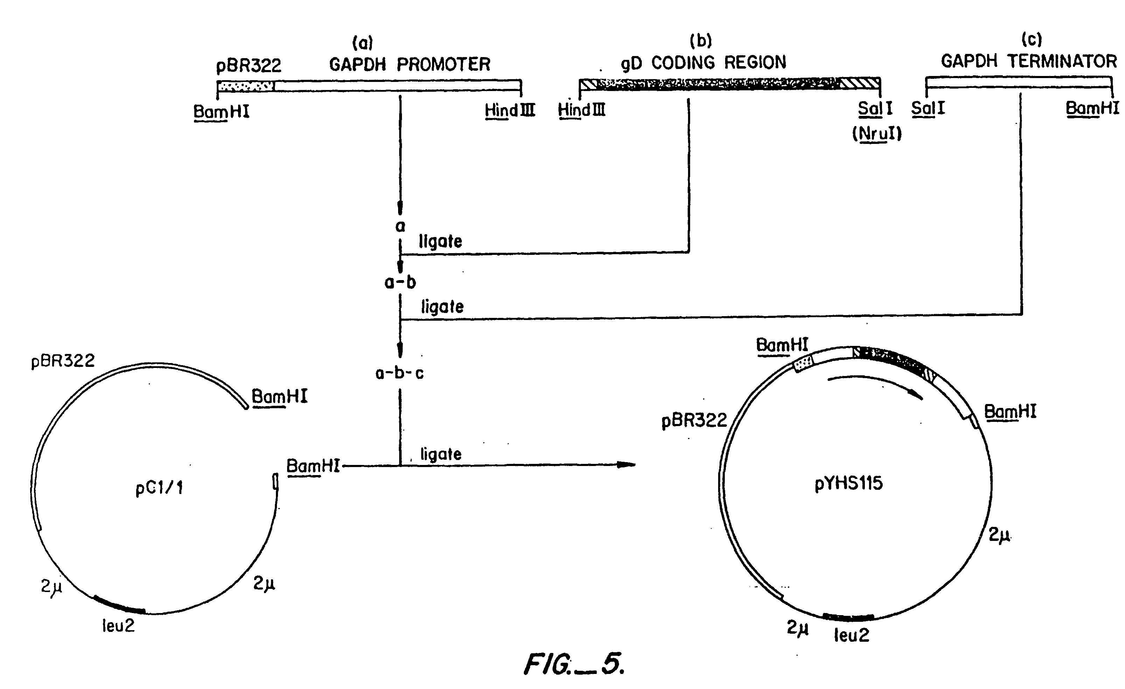

Fig. 5 represents the construction of plasmid pYHS115 which carries the naturally-occurring gD gene of HSV-1 under the transcriptional control of the GAPDH promoter and terminator, as described in the Experimental section hereinafter.

Fig. 6 is a restriction map for the entire HSV-1 genome.

Fig. 7 is a detailed restriction map of the subcloned region of the HSV-1 genome.

Fig. 8 is a restriction map of a 3.95kb BamHI-XhoI DNA fragment.

Fig. 9 is a restriction map for the HindIII H fragment of HSV-2 strain 333.

Fig. 10 is a detailed restriction map of the pHS203 XhoI subclone.

Fig. 11 is a restriction map of glycoprotein B of HSV-2.

## DESCRIPTION OF THE SPECIFIC EMBODIMENTS

### Vaccines

Vaccines employing recombinant Herpes Simplex Virus glycoprotein D of both Types 1 and 2 are provided. HSV gD may be used neat, but normally will be used in conjunction with a physiologically acceptable medium, generally water, saline, phosphate buffered saline, sugar, etc., and may be employed with a physiologically acceptable adjuvant, e.g. aluminum hydroxide. The vaccines may be administered by any convenient route, e.g. intravenously, intraarterially, subcutaneously, intramuscularly or intraperitoneally. It may be advantageous to administer split doses of vaccines which may be administered by the same or different routes.

Yeast produced Glycoproteins D may be used without further modification. However when smaller related polypeptides are used, such as fragments or the like and their molecular weight is less than 5000 daltons, and may be less then 1500 daltons, modification may be required to elicit the desired immune response. The smaller haptens should be conjugated to an appropriate immunogenic carrier such as tetanus toxoid or the like.

It may also be possible to link short DNA fragments encoding the gD polypeptide to genes expressing proteins from other pathogenic organisms or viruses. In this way, the resulting fused proteins may provide immunity for more than one disease.

The amount of glycoprotein or related polypeptide employed per dose will usually be about 10μg to 2mg/Kg, more usually about 50μg to 1mg/Kg and particularly about 100 to 500μg/Kg of host body weight. When two sub-units are employed, such as gB and gD, the ratio will usually be about 0.1 to 10:1, more usually about 0.5 to 10:1 and particularly about 0.5 to 5:1. The dose may be administered repeatedly at two to four week intervals, usually not more than about two to three times.

### Glycoprotein B

Examples concerned with gB are given for illustrative purposes only. Table 1 in the Experimental section provides the nucleotide sequence for gB1 strain Patton, as well as the amino acid sequence coded by the nucleotide sequence, while Table 2 gives a partial sequence demonstrating the substantial homology between gB1 and gB2. The nucleotide sequence may be varied in numerous ways. Various fragments may be employed having independent functions, which may be joined to proteins other than the mature gB. In addition, the various codons may be modified so as to encode for the same amino acids, but provide more efficient expression in accordance with the nature of the host. For example, the codons may be modified in accordance with the frequency of occurrence of a particular codon in one or more proteins or groups of proteins, e.g. glycolytic proteins, which contribute to a high proportion of the total proteins of a particular host, e.g., yeast. In some instances one or more codons may be modified to code for a different amino acid, substituting one amino acid for another amino acid, where the effect of the change is not detrimental to the immunogenicity of the protein or to other biological factors of interest. It may be desirable in some instances to add amino acids to the N-terminus or C-terminus, where such additional amino acids may provide for a desired result. This can be readily achieved by providing for additional codons at the 5'- or 3'-termini of the sequence encoding the mature gB1 or its precursor. In addition, while the amino acid sequence of gB2 may differ from that of gB1 by as much as 20 number percent, other strains of HSV-1 or of HSV-2 will have gB glycoproteins the same as or similar to gB1 strain Patton or gB2 strain 333, respectively, usually differing by fewer than 5 number percent, more usually differing by fewer than 2 number percent, and frequently differing by fewer than 0.5 number percent amino acids from the amino acid sequence of gB1 strain Patton or gB2 strain 333.

The gB1 sequence, particularly gB1 strain Patton, may be divided into four domains beginning at the N-terminus of the protein: A first hydrophobic region extending from amino acid 1 to about amino acid 56 to 60; a region of variable polarity extending from the first hydrophobic region to about amino acid 750 to 755, particularly 755; a second hydrophobic region extending from said variable polarity region to about amino acid 794 to 800, particularly 794, and a second variable polarity region extending to the C-terminus at amino acid 903.

Since gB is a membrane glycoprotein, based on analogy with other glycoproteins, the first hydrophobic region may be considered the signal leader sequence directing secretion and/or membrane location, particularly since it is followed by a dipeptide arginine arginine at amino acids 57 and 58, as well as 61 and 62, where the presence of two basic amino acids is known to be a peptidase signal for cleavage. The first sequence of variable polarity would then be external to the membrane and serve as the recognition sequence, to the extent that gB serves as a receptor for another protein or as an immunogen in a vaccine. The second hydrophobic sequence may serve as a transmembrane integrator sequence (often termed the "anchor"), which can be joined to other amino acid sequences to bind them to a membrane. The second variable polarity amino acid sequence would be expected to be in the cytoplasm and to the extent that a receptor is external to the transmembrane integrator sequence may serve to modulate one or more cytoplasmic processes.

Each of the above sequences may have individual utility as well as utility combined with the other sequences. The first hydrophobic sequence can serve as a signal leader sequence and be joined to DNA sequences coding for unnatural amino acid sequences to provide for transfer from the endoplasmic reticulum through the external membrane, with loss of the signal leader. (By "unnatural" is intended a sequence other than the naturally occurring sequence associated with an indicated flanking sequence.) In this manner, various proteins, particularly those produced in mammalian host cells can be modified with the signal leader sequence, so as to be secreted from the host cell. The first variable polar region can serve as an immunogen for the production of antibodies capable of neutralizing HSV, as a competitive inhibitor for infection, as a reagent in immunoassays, either labeled or unlabeled, for the detection of antibodies specific for gB, or the like; the second hydrophobic region can serve to bind a protein, i.e., "anchor" it, to a membrane. Thus, this region can be joined to unnatural proteins, so as to maintain the proteins bound to the membrane. By employing hybrid DNA technology, one can produce a wide variety of surface membrane proteins different from the naturally occurring surface membrane proteins. By inserting an unnatural polynucleotide sequence between the first and second hydrophobic regions, and expressing the hybrid polynucleotide in an appropriate host, the unnatural protein can be provided on the surface of the cell. Thus, cells can be modified as to their binding characteristics for use as reagents in assays, to modify their growth patterns, to act as competitive activators or inhibitors in vivo, and the like. Finally, the second hydrophobic region can be used in conjunction with the transmembrane integrator sequence and sequences encoding an unnatural polypeptide, where such polypeptide can serve as a receptor and actuate the second variable region to initiate a physiological effect in a host cell. In this manner, cells can be modified to respond to different ligands, as if binding was occurring to the gB protein.

It is evident from the above that the entire sequence or fragments thereof having functional significance can be employed in a variety of ways. The amino acid sequence or fragments thereof can be used by themselves or in combination with various labels in immunoassays, bioassays or the like, for the detection of HSV or antibodies to HSV, as immunogens, in vaccines, etc. Labels include radionuclides, enzymes, fluorescers, chemiluminescers, enzyme substrates or cofactors, enzyme inhibitors, particles, dyes, etc. These conjugates may be used in any convenient assay, such as enzyme immunoassays, e.g., ELISA, homogeneous enzyme immunoassays, fluorescence immunoassays, and the like. See, for example, U.S. Patent Nos. 3,766,162; 3,791,932; 3,817,837; 3,996,345 and 4,233,402.

The polynucleotide sequence encoding for the precursor to gB or functional fragments thereof may be cloned and expressed by inserting the polynucleotide sequence into an appropriate expression vector and introducing the resulting expression product construct into a compatible host. The coding fragments will be less than about 0.1 map unit, usually less than about 0.05 map unit. The expression vector may be a low or high multicopy vector and may provide for secretion or excretion of the polypeptide of interest or retention of the polypeptide of interest in the cytoplasm or in the membrane. A large number of cloning and expression vectors have been published in the literature and are generally available for use in either prokaryotic or eukaryotic hosts, including bacteria, e.g., E. coli, B. subtilis, etc., fungi, particularly yeast, e.g., S. cerevisiae, and a wide variety of immortalized mammalian cells, such as mouse cells, monkey cells, hamster cells, e.g., 3T3, Vero, Chinese Hamster Ovary cells (CHO), etc. Depending upon the host, where secretion is desired, either the native or unnatural secretory leader sequence may be employed. The processing signals for cleavage of the secretory leader may be the natural signals or the signals associated with the unnatural secretory leader or both in tandem. In some instances, the vector may result in integration into the genome of the host.

For secretion, the polynucleotide sequence will require the first hydrophobic region, while being free of the second hydrophobic region and normally the second variable polar region. For retention in the cytoplasm, the polynucleotide sequence will be free of the sequence coding the secretory leader sequence and may or may not have the second hydrophobic sequence including the second variable polar region. To remove the individual functional sequences or to join a particular functional sequence to an unnatural sequence, the polynucleotide sequence encoding the gB precursor can be restriction mapped and where appropriate restriction sites are found, the sequence may be cleaved at or near the desired site. Where excess nucleotides exist, these can be removed by resection, for example with Bal31 or where the sequence has been truncated, the lost nucleotides may be replaced by employing chemically synthesized adapters. By appropriate choice of adapters, restriction sequences may be maintained and/or new restriction sequences introduced. In some instances it may be useful to fill in the overhang resulting from restriction employing the Klenow fragment to provide for blunt ends, followed by blunt-end ligation with an appropriate ligase. Selection may then be made for the hybrid DNA having the appropriate orientation.

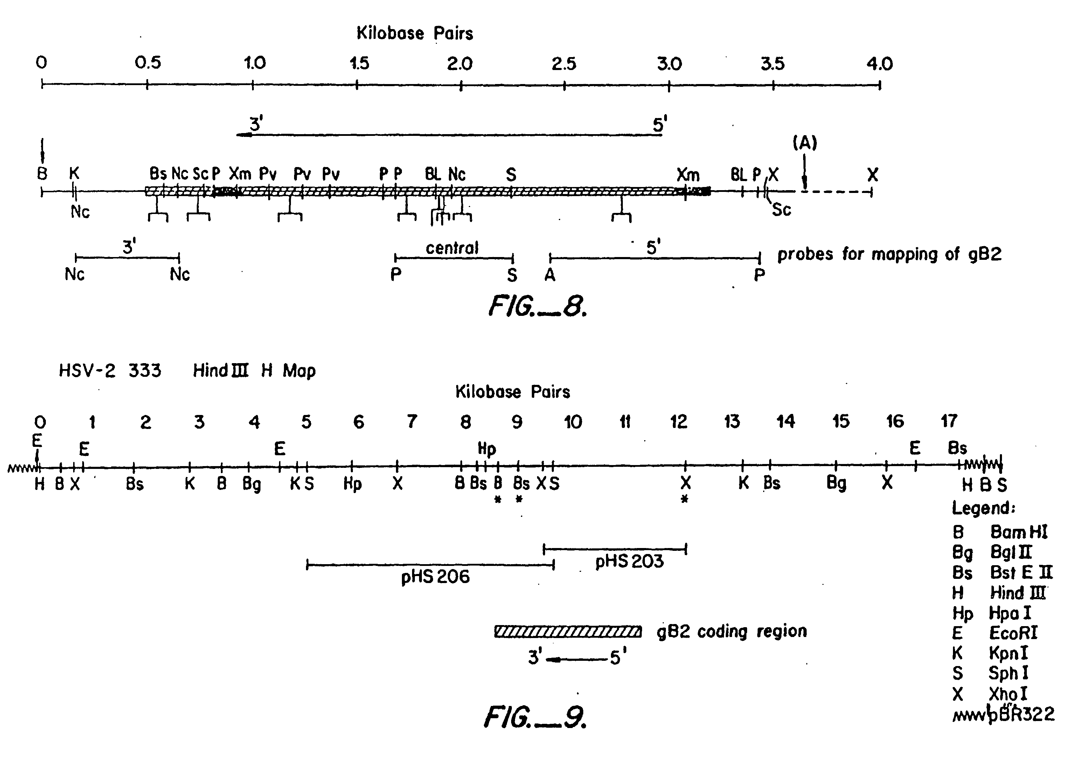

In order to obtain the polynucleotide sequence encoding for gBl-Patton, the location of the gB1 coding sequences on the EcoRI restriction fragment F was mapped. Three subfragments of the F fragment were isolated and subcloned into pBR322 (Fig. 7). DNA fragments from these subclones were then used to probe Northern blots of Poly A$^+$ mRNA isolated from HSV-1 infected cells. Fragments which hybridized to mRNA of the size expected for gB were presumed to lie within the gB coding region. The direction of transcription of gB was also elicited by determining which strand of the DNA probes hybridized with the mRNA. To verify the identity of the gB sequence, DNA fragments were used to hybrid-select HSV-1 mRNA, which was then translated in vitro and the resulting proteins analyzed for gB using a gB specific antibody.

The gB1 coding fragment may now be manipulated in a variety of ways, including restriction mapping and sequencing, so as to establish the restriction sites and the open reading frame regions for expression. The DNA sequence may then be restricted to provide for a sequence encoding the entire gB precursor or fragments thereof. These sequences may then be inserted into an appropriate expression vector having appropriately positioned transcriptional and, as appropriate, translational signals. As previously indicated, this can be achieved by filling in overhangs and providing for blunt-end ligation, by employing adapters, or the like.

Of particular interest is to introduce the gene in tandem with a gene capable of amplification. Convenient genes include the dihydrofolate reductase (DHFR) gene, which can be amplified by employing methotrexate, where the DHFR gene and flanking regions are reiterated; metallothioneins which can be amplified with heavy metals, e.g., copper, or the like. The expression product construct can be introduced into an appropriate host by any convenient means, including transformation, transfection, calcium phosphate precipitation, etc. The host cells may then be stressed with the appropriate biocide at levels which select for amplification of the particular gene. The cells may then be cultured and grown to provide efficient production of the desired polypeptide.

Following the procedure described above, the polynucleotide sequence coding for gB2-333, both precursor and mature, may be isolated, cloned, and manipulated to provide a construct which may result in expression in one or more hosts. However, in view of the availability of fragments coding for gB1-Patton, these fragments may be used as probes for either localization of gB2 encoding DNA segments to specific HSV-2 restriction fragment clone(s) or isolation of gB2 mRNA from infected host cells. Conveniently, a plurality of probes may be employed coding for different regions of the gB1 gene. One selects for either positive DNA fragments(s) or abundant mRNA having approximately the right size which hybridizes to the probe(s). The mRNA may then be reverse transcribed to provide cDNA or may be used for hybridization to fragments of the HSV-2 genome to confirm their gB2 encoding function.

### Glycoprotein D

Polypeptides which are immunologically cross-reactive with naturally-occurring glycoprotein D are produced in yeast by recombinant DNA methodology. Production in yeast allows the advantages associated with eukaryotic hosts, e.g., post-translational modification and secretion. The polypeptides of the present invention may be produced from relatively short synthetic DNA fragments encoding for at least about nine amino acids to provide haptens useful for eliciting an immune response specific for gD, e.g., for use in vaccine production. The cloned DNA fragments (or the RNA produced therefrom) may also find use as hybridization probes in a variety of applications. Alternatively, much longer DNA fragments, either synthetic or natural, may be used to produce larger polypeptides for use as vaccines, immunogens, immunological reagents, and the like. The peptides of the present invention will find particular use in the preparation of vaccines against infection by Herpes Simplex Virus.

The glycoprotein D (gD) DNA fragments of the present invention may be of natural or synthetic origins. The natural gD gene of HSV-1 is located on the viral genome between the short internal repeat (IR$_S$) sequence and short terminal repeat (TR$_S$) sequence at the 3'-end thereof. Coding for the mature protein is found on an approximately 1.6kbp fragment located on a 2.9kbp SacI restriction fragment of the genome. The entire coding region for the mature protein is located within a HindIII-NruI fragment of the 2.9kbp SacI fragment. The naturally-occurring gD gene may be employed with or without modification. Regions of the gene may be deleted and/or joined to other DNA fragments as desired. The preparation, cloning and expression of particular fragments of the naturally-occurring gD gene are described in detail in the Experimental section hereinafter.

Synthetic gD sequences will also find use, either alone or in combination with the naturally-occurring sequences. Coding for the synthetic sequences may be based on published nucleotide sequences for gD found in the literature. See, Watson et al., (1982), supra. The synthetic fragments will be sufficiently long to code for at least nine contiguous amino acids with a maximum length equal to that of the naturally-occurring gene or longer. When preparing polypeptides for use as immunological reagents or as vaccines, it is usually desirable that the nucleotide sequence code for an oligopeptide corresponding to an epitopic site of the natural gD protein. Often, such epitopic sites may be predicted by employing the folding rules of Chou and Fasman, Biochemistry (1974) 13:211-222, in conjunction with an analysis of hydrophobic and hydrophilic regions of the protein (Hopp and Woods, Proc. Natl. Acad. Sci. USA (1981) 78: 3824-3828). In particular, it has been found that polypeptides corresponding to amino acids 253-283 and 8-23 in the mature naturally-occurring gD protein will cross-react with antisera raised against the natural gD protein.

A number of techniques are available for synthesizing short single-stranded DNA fragments, e.g., the phosphoramidite method described by Beaucage and Carruthers, Tetrahedron Lett. (1981) 22:1859-1862. Short fragments having a length of about 40 bases or less may be synthesized as a continuous single-stranded fragment. The complementary strand may also be synthesized, and the two strands annealed under appropriate conditions. Alternatively, the complementary strand may be added using DNA polymerase with an appropriate primer sequence.

The synthesis of longer fragments exceeding 100 base pairs is typically accomplished by the preparation of a series of single-stranded DNA fragments including from about 10 to 100 bases, usually from about 15 to 60 bases. The sequences of such fragments are selected so that when the fragments are brought together under annealing conditions, a double stranded fragment having the desired nucleotide sequence is produced. When devising such synthetic nucleotide sequences, the sequences of the individual single stranded DNA fragments are examined to assure that annealing of unmatched segments is avoided. In this way, base pairing occurs only between those segments which are intended to be annealed in the resulting double stranded DNA fragment. Undesired base pairing may usually be avoided by altering one nucleotide (usually the third nucleotide) in preselected ones of the codons.

When preparing synthetic gD DNA fragments, it may sometimes be desirable to modify the reported nucleotide sequence. For example, it will usually be preferred to use codons which are preferentially recognized by the intended yeast host. Specifically, such codons are those which appear at high frequency in the structural genes encoding for the yeast glycolytic enzymes. The nucleotide sequence will usually comprise at least 50% preferred yeast codons, more usually at least 60%, and preferably at least 75%. In some instances, it may be desirable to further alter the nucleotide sequence to create or remove restriction sites or to substitute one or more amino acids in the resulting polypeptide. Such changes may be made to enhance the immunogenicity of the polypeptide, facilitate conjugating the polypeptide to a carrier protein, or the like, etc. It may also be desirable at times to add amino acids to the N-terminus or C-terminus of the polypeptide, where such additional amino acids may provide for a desired result.

The DNA fragments coding for a desired gD fragment will be incorporated in DNA constructs capable of self-replication and expression in yeast. Such DNA constructs will include a replication system recognized by the yeast host, the gD DNA fragment encoding the desired polypeptide product, transcriptional and translational initiation regulatory sequences joined to the 5'-end of the gD sequence, and transcriptional and translational termination regulatory sequences joined to the 3'-end of the gD sequence.

The transcriptional regulatory sequences will include a promoter, which may be the promoter associated with the secretory leader and processing signal sequence. Other promoters may also find use, particularly those involved with the enzymes in a yeast glycolytic pathway, such as the promoters for alcohol dehydrogenase, glyceraldehyde-3-phosphate dehydrogenase (GAPDH), pyruvate kinase, triose phosphate isomerase, phosphoglucoisomerase, phosphofructokinase, and the like. By employing these promoters with other regulatory sequences, such an enhancers, operators, and the like, and using a host having an intact regulatory system, one can regulate the expression of the gD polypeptide product by varying the carbon source, e.g., replacing glucose with galactose; varying the concentration of a nutrient, e.g., phosphate, or changing the temperature with a temperature sensitive promotor or regulatory system.

The transcriptional termination regulatory sequence will include a terminator, preferably a terminator balanced with the promoter to provide for proper transcription. Conveniently, the terminator which is naturally found with the promoter may be employed. The remaining sequences in the construct, including the replication systems for both yeast, e.g., 2μ plasmid, and bacteria, e.g., Col E1, are well known and amply described in the literature.

Enhanced yields of shorter polypeptides may be obtained by employing DNA constructs which include a secretory leader and processing signal sequence to effect secretion and post-translational modification of the gene product in yeast. The upper size limit on the secreted polypeptides is not fixed, although they will usually be below 40 kilodaltons, more usually below about 30 kilodaltons. Moreover, size is not the only determinative criteria as hydrophobic peptides may be more readily secreted than non-hydrophobic peptides. The secretory leader and processing signal sequences will normally be derived from naturally-occurring DNA sequences in yeast which provide for secretion of a polypeptide. Such polypeptides which are naturally secreted by yeast include α-factor, a-factor, acid phosphatase, and the like. If desired, the naturally-occurring sequence may be modified, for example, by reducing the number of lys-arg pairs which define the processing site (while retaining at least one pair), or by reducing the length of the secretory leader sequence (while retaining sufficient length to provide for secretion), or by introducing point mutations, deletions or other modifications which facilitate manipulation, e.g., introducing restriction recognition sites. Conveniently, the secretory leader and processing signal sequence may be joined to the gD DNA fragment by providing appropriate cohesive ends on the gD fragment, by use of appropriate adaptor molecules, or a combination of both.

Polypeptides of the present invention may also be produced intracellularly as follows. After the transformed cell culture has reached a high density, the cells will be separated, typically by centrifugation, lysed, and the gD polypeptides isolated by various techniques, such as extraction, affinity chromatography, electrophoresis, dialysis and combinations thereof.

The polypeptides of the present invention, and fragments thereof, may be employed in a variety of ways. The polypeptides can be employed both as labelled and unlabelled reagents in various immunoassays, bioassays, and the like, for the detection of HSV or anti-bodies to HSV. Suitable labels include radionuclides, enzymes, fluorescers, chemiluminescers, enzyme substrates or co-factors, enzyme inhibitors, particles, dyes, and the like. Such labelled reagents may be used in a variety of well known assays, such as radioimmunoassays, enzyme immunoassays, e.g., ELISA, fluorescent immunoassays, and the like. See, for example, U.S. Patent Nos. 3,766,162; 3,791,932; 3,817,837; 3,996,345; and 4,233,402.

The following examples are offered by way of illustration and not by way of limitation.

EXPERIMENTAL

Glycoprotein B

Materials and Methods.

The HSV-1 strain Patton and HSV-2 strain 333 viable stocks are available from Dr. Richard Hyman, Hershey Medical Center, Hershey, Pennsylvania. These viruses can be propagated in Vero cells available from Dr. Evelyn Linnette, Viro Labs, Emeryville, California, or from the American Type Tissue Culture Laboratory, the propagation being performed in accordance with standard procedures. A library of HSV-1 Patton EcoRI DNA fragments cloned in the EcoRI site of the plasmid pACYC184 (Chang and Cohen, J. Bacteriology (1978) 134:1141) can be obtained from Dr. Hyman or be independently prepared in accordance with conventional techniques. Two HSV-2 333 clones can also be obtained from Dr. Hyman, namely the HindIII fragments H and L inserted into the HindIII site of pBR322 (Sutcliffe, Nucleic Acids Research (1978) 5:2721).

Subcloning.

Restriction enzymes, T4DNA ligase, E. coli DNA polymerase I Klenow fragment, and other biological reagents were purchased from Bethesda Research Laboratories or other indicated commercial suppliers and used according to the manufacturer's directions. Double-strand DNA fragments were separated on 1% agarose gels and isolated by electroelution.

Isolation of RNA, Northern blot analysis and hybrid-selected translation.

Total RNA was prepared from HSV-1 infected Vero cells at 6 hrs after infection. Cell monolayers were washed, incubated with extraction buffer and processed as described (Pachl et al., Cell (1983) 33:335-344). Poly A$^+$ RNA was prepared by passing total RNA over a 3ml column of oligo dT cellulose (obtained from Collaborative Research) in 500mM NaCl, 10mM Tris HCl pH 7.5, 1mM EDTA, 0.1% SDS, then washing the column in 100mM NaCl, 10mM Tris HCl pH 7.5, 1mM EDTA, 0.1% SDS and then eluting the poly A$^+$ with 10mM Tris HCl pH 7.5, 1mM EDTA, 0.1% SDS.

For Northern blot analysis, poly A$^+$ RNA was denatured with glyoxal (McMaster et al., Proc. Natl. Acad. Sci. USA (1977) 74:4835-4838, fractionated by electrophoresis on 1% agarose gels, transferred to nitrocellulose paper (Thomas, ibid. (1980) 77:5201-5205) and hybridized with $^{32}$P-labeled probes.

The details of the methods used for hybrid-selected translation have been described previously (Pachl et al., Cell (1983) 33:335-344). DNA filters were prepared using either 3μg of a 3.5kb Xho-Kpn fragment encoding gB or 2μg of a 3.0kb SstI-SstI fragment encoding HSV-1 glycoprotein gD. The filters were incubated with 40μg of poly A$^+$ RNA from HSV-1 infected cells. Bound RNA was eluted and translated in a reticulocyte cell-free system (Pachl et al., J. Virol. (1983) 45:133-139). Translation products were analyzed on 12.5% sodium dodecyl sulfate (SDS) polyacrylamide gels (Laemmli, Nature (1970) 227:680).

In vivo labeling of cells and immunoprecipitation.

To label with $^{35}$S-methionine, cells were grown to confluency in 3.5cm dishes, washed once with PBS (0.14M NaCl, 2.7mM KCl, 15.3mM Na$_2$HPO$_4$, 1.5mM KH$_2$PO$_4$) and then 0.5ml of labeling media, DME (Dulbecco's Modified Eagle medium from Gibco, cat. no. 188G) without methionine plus 1% dialyzed fetal calf serum and 400μCi/ml $^{35}$S-

methionine (>1000Ci/mmole) was added per dish. The cells were incubated for appropriate times at 37°C. At the end of the labeling period, the media was removed and the monolayer washed once with PBS. For a "cold" methionine chase, the labeling media was replaced with DME containing 2.5mM methionine. For immune precipitation, cells were lysed in 0.1ml of lysis buffer: 20mM Tris-HCl pH8, 100mM NaCl, 1mM EDTA, 0.5% Nonidet P40, 0.5% sodium deoxycholate, bovine serum albumin, 0.1% SDS, 1.0mM phenylmethylsulfonyl fluoride, 10mM benzamidine, 1% aprotenin obtained from Sigma Chemical Company. The cell lysate was scraped into tubes, briefly vortexed, and then held at 4°C for 5-10min. Cell debris was removed by centrifugation and the clarified lysate stored at -70°C.

For immunoprecipitations, cell lysates, 0.1ml, were precleared by incubation with normal serum for 30min at 4°C, then 50µl of a 20% solution of protein A Sepharose (PAS) (in lysis buffer) was added and incubation continued for 30min at 4°C with gentle rocking. The PAS was removed by centrifugation for 1min at 14,000xg and 5µl of HSV-1 polyclonal antibody (obtained from DAKO) or a gB-specific monoclonal antibody F3AB (obtained from Dr. John Oakes, University of South Alabama) was added. When the F3AB antibody was used, 0.1% SDS was omitted from the lysis buffer. After 30min at 4°C, 75µl of PAS was added and incubated as above. PAS-immune complexes were collected by centrifugation, washed 3x with lysis buffer lacking BSA and protease inhibitors and once with 0.12M Tris HCl pH 7.0. Immune precipitated proteins were released from PAS by boiling in SDS sample buffer, followed by analysis on 12% polyacrylamide gels. For immune precipitation of labeled proteins from cell media, the media was first clarified by centrifugation and then 1/10 volume of 10x lysis buffer was added and proteins were precipitated as described above.

Immunofluorescence.

To analyze gB synthesis in CHO clones, cells, grown in slide wells, were washed 3x with PBS, fixed with 100% methanol at -20°C for 10min followed by 3 more PBS washes and one wash with PBS plus 5% goat serum (GS). The fixed cells were then incubated with the primary antibody, HSV-1 polyclonal diluted 1/100 in PBS-5% GS, for 30min at 37°C. The cells were then washed 3x in PBS-5% GS and then incubated at 37°C for 30min with the second antibody, FITC-conjugated goat anti-rabbit IgG (Cappel), diluted 1/10 in PBS-5% GS. After 4 washes in PBS-5% GS, the slides were mounted with coverslips using 50% glycerol--100mM Tris HCl, pH 8 and observed in a Leitz microscope equipped with epifluorescent optics. Live cell immunofluorescence was carried out as described above except that the cells were initially washed once in PBS-5% GS directly followed by incubation with the first antibody. Before mounting with coverslips, the live cells were fixed with 5% formaldehyde in PBS. The fluorescein stained cells were photographed using Kodak Ektachrome film (ASA 400).

Cells and tissue culture.

The dhfr deficient CHO cell line was obtained from Dr. Y.W. Kan, (University of California at San Francisco). This cell line was originally described by Urlaub and Chasin, Proc. Natl. Acad. Sci. USA (1980) 77:4216-4220. For non-selective conditions, these cells were grown in Ham's F-12 medium (available from Gibco, cat. no. 176) supplemented with 10% fetal calf serum, 100U/ml penicillin, 100µg/ml streptomycin and 150µg/ml L-proline. Selective media was DME supplemented with 10% dialyzed fetal calf serum plus penicillin, streptomycin and 150µg/ml L-proline. For methotrexate (MTX) selection, concentrated MTX stocks were prepared from MTX obtained from Lederle and added to the above DME selective media immediately before use.

DNA transfections.

Transformation of dhfr deficient CHO cells with plasmids pSV1/dhfr, pHS112 and pHS114 was carried out using the procedure of van der Eb and Graham (Methods in Enz. (1980) 65:826-839), as modified by Parker and Stark, (J. of Virol. (1979) 31:360-369), except that carrier DNA was omitted. A calcium phosphate precipitate of plasmid DNA was prepared by mixing an equal volume of plasmid DNA, in 250mM $CaCl_2$, with an equal volume of 2x concentrated HEPES-buffered saline (2xHBS) added dropwise (1xHBS is 0.14M NaCl, 5mM KCl, 0.7mM $Na_2HPO_4$, 2.8mM glucose, 10mM HEPES pH 7.0). After about 20min incubation at room temperature, 1ml of the calcium-phosphate-DNA suspension (containing 15µg DNA) was added to the media of cells, grown to 50% confluency on 10cm plates. After 6-8 hrs the DNA containing media was removed and the cells were incubated with 15% glycerol-IxHBS for 4min. The cells were then grown in non-selective media (F12) for two days, after which the cells were split, i.e., subcultured, into selective media. Colonies of dhfr positive cells appeared after 10 days and were isolated after 14 days by removing the cells of a colony from a dish with a Pasteur pipette. The isolated cells were transferred to multiwell dishes for propagation.

Isolation, cloning and characterization of the gB1 gene.

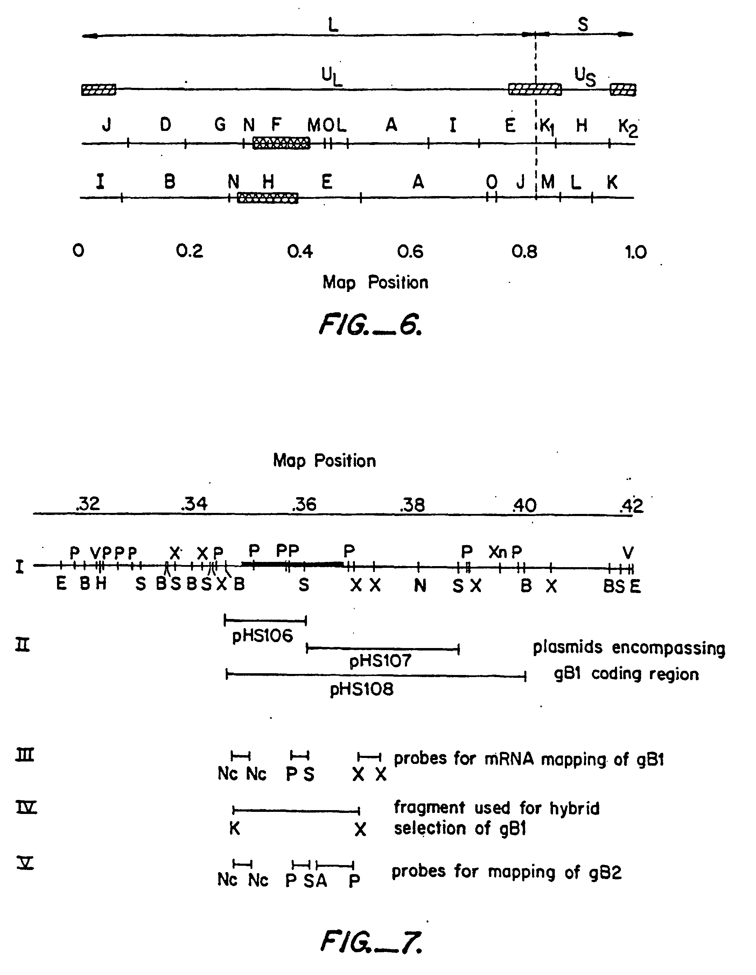

To isolate the gene for the glycoprotein gB1, DNA fragments spanning map coordinates 0.345 to 0.40 within the EcoRI F restriction fragment of the HSV-1 strain Patton (Skare and Summers, Virology (1977) 76:581-595) were subcloned in the plasmid pBR322. These fragments were prepared from the appropriate restriction digests of the EcoRI F region in the plasmid pACYC184, separated by electrophoresis on a 1% agarose gel in TAE buffer (0.04M Tris-acetate, 0.002M EDTA) and electroeluted as noted above. The isolated fragments were ligated into pBR322 which had also been previously cut with the appropriate restriction enzyme and treated with alkaline phosphatase. A restriction map for the entire HSV-1 genome is shown in Fig. 6, and a more detailed map of the region which was subcloned is shown in Fig. 7. Referring to Fig. 6, the conventional map is shown in the first two lines (Roizman, 1979). The dotted line indicates the L-S junction. The restriction enzyme cleavage map for EcoRI for the prototype isomer arrangement is shown in the third line (Skare and Summers, 1977; Roizman, 1979) with the EcoRI fragment F denoted by the cross-hatched box. For HSV-2, the HindIII restriction map is shown in line 4 (Roizman, 1979) with the HindIII fragment H cross-hatched. One map unit corresponds to 98.9 megadaltons or 148.9kbp of DNA for HSV-1 and 105.6 megadaltons or 160.5kbp of DNA for HSV-2.

Referring to Fig. 7, the restriction enzyme sites shown in the detailed map line (I) are E, EcoRI; B, BamHI; S, SalI; P, PstI; X, XhoI from DeLucca et al., 1983; N, NdeI; Xn, XmnI; V, EcoRV. The BstEII site mapped by DeLucca et al. at 0.355 is missing in this strain and there is a new PstI site at 0.357. Line II shows three plasmid subclones which encompass the gB1 coding region. They are pHS106, which extends from the BamHI site at 0.345 to the SalI site at 0.360; pHS107 which extends from the SalI site at 0.36 to the SalI site at 0.388; and pHS108 which is a BamHI fragment extending from 0.345 to 0.40 map units. Line III indicates three probes used for mRNA mapping of gB1; line IV indicates the fragment used for hybrid selection; and line V shows those probes used to locate the gB2 gene (see below). The additional restriction sites used to generate these fragments are Nc, NcoI; K, KpnI; and A, AluI.

To locate the gB1 coding region within the EcoRI F fragment, Northern blots of poly A$^+$ mRNA isolated from HSV-1 infected Vero cells were probed with the DNA fragments indicated on the detailed map isolated from plasmids pHS106 and pHS107. When HSV-1 mRNA was probed with a 0.56kb PstI-SalI fragment isolated from pHS106, a 3kb mRNA was the major species detected. When the same blot was probed with a 0.49kb NcoI fragment, which maps about lkb upstream from the PstI-SalI fragment, hybridization to a 3kb mRNA, the presumptive gB1 mRNA was also detected. This suggests that the gB1 coding sequences extend at least lkb to the left of the PstI-SalI fragment. The 3kb mRNA does not extend beyond the first XhoI site downstream from the PstI-SalI fragment, since the 0.5kb XhoI-XhoI fragment does not hybridize to this mRNA. The direction of transcription of the gB1 transcription unit is right to left ($3' \leftarrow 5'$) as evidenced by hybridization of only the $5' \rightarrow 3'$ oriented strands of the PstI-SalI and NcoI-NcoI fragments (cloned in M13) to the 3kb gB1 mRNA.

Hybrid selected translation was performed by hybridizing HSV-1 poly A$^+$ mRNA with a 3.2kb KpnI-XhoI fragment, which encompasses the region indicated as encoding gB1. When the bound mRNA was eluted and translated in vitro, a 100kd protein, similar in size to gB1 from HSV-1 infected Vero cells, was detected. Confirmation of the identity of the 100kd protein was achieved by immunoprecipitation with a gBl-specific monoclonal antibody. Several other proteins were also detected by hybrid selection using the KpnI-XhoI fragment, probably the result of non-specific hybridization of mRNAs due to the high G+C content of the DNA. A similar pattern of proteins was seen when the same RNA was selected with a 3.0kb SstI-SstI DNA fragment encoding HSV-1 glycoprotein gD, except that the 100kd gB protein was not detected. This result indicates that gB is specific to the XhoI-KpnI fragment.

Fig. 8 is a restriction map of a 3.95kb DNA fragment, which extends from a BamHI restriction site at 0.345 to an XhoI site at 0.373 map units. The open reading frame for gB1 is indicated by the box and the direction of transcription is from right to left as shown. The actual coding region covers map units 0.348 to 0.367. The DNA sequence from the BamHI site to a non-unique AluI site at nucleotide number 3640 is shown with the AluI site indicated by the (A). The restriction sites shown include B, BamHI; Bl, BalI; Bs, BstEII; K, KpnI; Nc, NcoI; P, PstI; Pv, PvuII; S, SalI; Sc, SacI; X, XhoI; Xm, Xma3. Restriction sites are not shown for the right-hand end from the AluI site to the terminal XhoI site. Three probes used for mapping the gB2 gene and potential glycosylation sites and hydrophobic anchor and signal regions (solid box) in the product gB1 protein are noted.

The DNA sequence was determined from the BamHI site to a non-unique AluI site at nucleotide residue number 3640. The DNA sequence was determined using the M13 dideoxynucleotide synthesis method of Sanger from both DNA strands across the coding region. The DNA sequence was obtained from overlapping restriction fragments such that the sequence was read across all restriction fragment joints. The following Table 1 is the sequence for the above-indicated DNA fragment, with the derived amino acid sequence for gB1.

<u>Table 1</u>

```
AGCTCGCCCCGGTTCATCATGCGGGGCGGGGCGGCTCCCGTGCTGATTCGGCCGTCAGCGAA
                                                        50
TTTCACAGGTTTTACTGTTTTGACGGCATTTCCGGAATAACGCCCACTCAGCGGCGCCCGC
                                                        100
CTCGCCGATATATTCGCGAGCTGATTATCGCCACCACACTCTTTGCCTCGGTCTACCCGT
                                                150
GCGGGGAGCTCGAGTTGCGCCCGCCCCGGACTGCAGCCGCCCGACCTCCGAAGTCGTTACC
                                        200
GTTACCCGCCCGGCCTATATCTCACGTACGACTCCGACTGTCCGCTGGTGGCCATCGTC
                                250
GAGAGCGCCCCCGACGGCTGTATCGGCCCCCCGGTCGGTCGTGGTCTACGACCGAGACGT
                    300
TTTCTCGATCCTCTACTCGGTCCTCCAGCACCTCGCCCCCAGGCTACCTGACGGGCGCGC
                                                        400
```

```
                                        1
                                        met arg gln gly ala pro ala arg
ACGACGGGCCCCCGTAGTCCCGCC                ATG CGC CAG GGC GCC CCC GCG CGG
                                                        450
         10                                     20
gly cys arg trp phe val val trp ala leu leu gly leu thr leu
GGG TGC CGG TGG TTC GTC GTA TGG GCG CTC TTG GGG TTG ACG CTG
                                                        500
                        30
gly val leu val ala ser ala ala pro ser ser pro gly thr pro
GGG GTC CTG GTG GCG TCG GCG GCT CCG AGT TCC CCC GGC ACG CCT
                                                        550
         40                                     50
gly val ala ala ala thr gln ala ala asn gly gly pro ala thr
GGG GTC GCG GCC GCG ACC CAG GCG GCG AAC GGG GGC CCT GCC ACT

                                 60
pro ala pro arg arg pro trp arg arg pro asn gly gly pro glu
CCG GCG CCG CGC CGC CCT TGG CGC CGC CCC AAC GGG GGA CCC GAA
         600
         70                                     80
thr glu glu glu gln lys thr glu lys pro asn ala ala thr pro
ACC GAA GAA GAA CAA AAA ACC GAA AAA CCC AAC GCC GCC ACG CCC
                                 650
                                 90
arg arg arg gln arg asp arg arg arg gly pro arg his pro ala
CGC CGG CGA CAA CGC GAC CGT CGC CGC GGG CCA CGC CAC CCT GCG
                         700
         100                                     110
arg ala leu arg asp ile lys ala glu asn thr asp ala asn phe
CCA GCA CTC CGC GAC ATC AAG GCG GAG AAC ACC GAT GCA AAC TTT
                                 750
                                 120
tyr val cys pro pro pro thr gly ala thr val val gln phe glu
TAC GTG TGC CCA CCC CCC ACG GGC GCC ACG GTG GTG CAG TTC GAG
                                         800
         130                                     140
gln pro arg arg cys pro thr arg pro glu gly gln asn tyr thr
CAG CCG CGC CGC TGC CCG ACC CGG CCC GAG GGT CAG AAC TAC ACG
                                         850
```

```
                        150
glu gly ile ala val val phe lys glu asn ile ala pro tyr lys
GAG GGC ATC GCG GTG GTC TTC AAG GAG AAC ATC GCC CCG TAC AAG
                                                  900
       160                                170
phe lys ala thr met tyr tyr lys asp val thr val ser gln val
TTC AAG GCC ACC ATG TAC TAC AAA GAC GTC ACC GTT TCG CAG GTG
                                                      950
                              180
trp phe gly his arg tyr ser gln phe met gly ile phe glu asp
TGG TTC GGC CAC CGC TAC TCC CAG TTT ATG GGG ATC TTT GAG GAC
                                                      1000
       190                                200
arg ala pro val pro phe glu glu val ile asp lys ile asn ala
CGC GCC CCC GTC CCC TTC GAG GAG GTG ATC GAC AAG ATC AAC GCC

                        210
lys gly val cys arg ser thr ala lys tyr val arg asn asn leu
AAG GGG GTC TGT CGG TCC ACG GCC AAG TAC GTG CGC AAC AAC CTG
       1050
       220                                230
glu thr thr ala phe his arg asp asp his glu thr asp met glu
GAG ACC ACC GCG TTT CAC CGG GAC GAC CAC GAG ACC GAC ATG GAG
             1100
                              240
leu lys pro ala asn ala arg asp pro his glu pro gly leu ala
CTG AAA CCG GCC AAC GCC CGC GAC CCG CAC GAG CCG GGG CTG GCA
                        1150
       250                                260
his his arg pro gln val gln pro leu ala gly gly gly val pro
CAC CAC CGA CCT CAA GTA CAA CCC CTC GCG GGT GGA GGC GTT CCA
                        1200
                        270
pro val arg thr thr val asn cys ile val glu glu val asp ala
CCG GTA CGG ACG ACG GTA AAC TGC ATC GTC GAG GAG GTG GAC GCG
                        1250
       280                                290
arg ser val tyr pro tyr asp glu phe val leu ala thr gly asp
CGC TCG GTG TAC CCG TAC GAC GAG TTT GTG CTG GCG ACT GGC GAC
                                             1300
                        300
phe val tyr met ser pro phe tyr gly tyr arg glu gly ser his
TTT GTG TAC ATG TCC CCG TTT TAC GGC TAC CCG GAG GGG TCG CAC
                                             1350
       310                                320
thr glu his thr ser tyr ala ala asp arg phe lys gln val asp
ACC GAA CAC ACC AGC TAC GCC GCC GAC CGC TTC AAG CAG GTC GAC
                                                  1400
                        330
gly phe tyr ala arg asp leu thr thr lys ala arg ala thr ala
GGC TTC TAC GCG CGC GAC CTC ACC ACC AAG GCC CGG GCC ACG GCG
                                                  1450
       340                                350
pro thr thr arg asn leu leu thr thr pro lys phe thr val ala
CCG ACC ACC CGG AAC CTC CTC ACG ACC CCC AAG TTC ACC GTG GCC
```

11

```
                        360
trp asp trp val pro lys arg pro ser val cys thr met thr lys
TGG GAC TGG GTG CCA AAG CGC CCG TCG GTC TGC ACC ATG ACC AAG
        1500
    370                                          380
trp gln glu val asp glu met leu arg ser glu tyr gly gly ser
TGG CAG GAG GTG GAC GAG ATG CTG CGC TCC GAG TAC GGC GGC TCC
            1550
                        390
phe arg phe ser ser asp ala ile ser thr thr phe thr thr asn
TTC CGA TTC TCC TCC GAC GCC ATA TCC ACC ACC TTC ACC ACC AAC
                1600
    400                                          410
leu thr glu tyr pro leu ser arg val asp leu gly asp cys ile
CTG ACC GAG TAC CCG CTC TCG CGC GTT GAC CTG GGG GAC TGC ATC
                        1650
                        420
gly lys asp ala arg arg his gly pro his leu arg pro gln tyr
GGC AAG GAC GCC CGA CGC CAT GGA CCG CAT CTT CGC CCG CAG TAC
                            1700
    430                                          440
asn ala thr his ile lys val gly gln pro gln tyr tyr leu ala
AAC GCG ACG CAC ATC AAG GTG GGC CAG CCG CAG TAC TAC CTG GCC
                                    1750
                        450
asn gly gly phe leu ile ala tyr gln pro leu leu ser asn thr
AAT GGG GGC TTT CTG ATC GCG TAC CAG CCC CTT CTC AGC AAC ACG
                                        1800
    460                                          470
leu ala glu leu tyr val arg glu his leu arg glu gln ser arg
CTC GCG GAG CTG TAC GTG CGG GAA CAC CTC CGA GAG CAG AGC CGC
                                            1850
                        480
lys pro pro asn pro thr pro pro pro pro gly ala ser ala asn
AAG CCC CCA AAC CCC ACG CCC CCG CCG CCC GGG GCC AGC GCC AAC
                                                1900
    490                                          500
ala ser val glu arg ile lys thr thr ser ser ile glu phe ala
GCG TCC GTG GAG CGC ATC AAG ACC ACC TCC TCC ATC GAG TTC GCC

                        510
arg leu gln phe thr tyr asn his ile gln arg his val asn asp
CGG CTG CAG TTT ACG TAC AAC CAC ATA CAG CGC CAT GTC AAC GAT
        1950
    520                                          530
met leu gly arg val ala ile ala trp cys glu leu gln asn his
ATG TTG GGC CGC GTT GCC ATC GCG TGG TGC GAG CTG CAG AAT CAC
            2000
                        540
lys leu thr leu trp asn glu ala arg lys leu asn pro asn ala
AAG CTG ACC CTG TGG AAC GAG GCC CGC AAG CTG AAC CCC AAC GCC
                2050
    550                                          560
ile ala ser ala thr val gly arg arg val ser ala arg met leu
ATC GCC TCG GCC ACC GTG GGC CGC CGG GTG AGC GCG CGG ATG CTC
                        2100
```

```
                             570
gly asp val met ala val ser thr cys val pro val ala ala asp
GGC GAC GTG ATG GCC GTC TCC ACG TGC GTG CCG GTC GCC GCG GAC
                                        2150
     580                                             590
asn val ile val gln asn ser met arg ile ser ser arg pro gly
AAC GTG ATC GTC CAA AAC TCG ATG CGC ATC AGC TCG CCG CCC CGG
                                        2200
                             600
ala cys tyr ser arg pro leu val ser phe arg tyr glu asp gln
GCC TGC TAC AGC CGC CCC CTG GTC AGC TTT CGG TAC GAA GAC CAG
                                                     2250
     610                                             620
gly pro leu val glu gly gln leu gly glu asn asn glu leu arg
GGC CCG TTG GTC GAG GGG CAG CTG GGG GAG AAC AAC GAG CTG CGG
                                                          2300
                             630
leu thr arg asp ala ile glu pro cys thr val gly his arg arg
CTG ACG CGC GAT GCG ATC GAG CCG TGC ACC GTG GCA CAC CGG CGC
                                                             2350
     640                                             650
tyr phe thr phe gly gly gly tyr val tyr phe glu glu ser ala
TAC TTC ACC TTC GGT GGG GGC TAC GTG TAC TTC GAG GAG TCA GCG
                             660
tyr ser his gln leu ser arg ala asp ile thr thr val ser thr
TAC TCC CAC CAG CTG AGC CGC GCC GAC ATC ACC ACC GTC AGC ACC
     2400
     670                                             680
phe ile asp leu asn ile thr met leu glu asp his glu phe val
TTC ATC GAC CTC AAC ATC ACC ATG CTG GAG GAT CAC GAG TTT GTC
                2450
                             690
pro leu glu val tyr thr arg his glu ile lys asp ser gly leu
CCC CTG GAG GTG TAC ACC CGC CAC GAG ATC AAG GAC AGC GGC CTG
                2500
     700                                             710
leu asp tyr thr glu val gln arg arg asn gln leu his asp leu
CTG GAC TAC ACG GAG GTC CAG CGC CGC AAC CAG CTG CAC GAC CTG
                             2550
                             720
arg phe ala asp ile asp thr val ile his ala asp ala asn ala
CGC TTC GCC GAC ATC GAC ACG GTC ATC CAC GCC GAC GCC AAC GCC
                             2600
     730                                             740
ala met phe ala gly leu gly ala phe phe glu gly met gly asp
GCC ATG TTC GCG GGC CTG GGT GCG TTT TTC GAG GGG ATG GGC GAC
                                                     2650
                             750
leu gly arg ala val gly lys val val met gly ile val gly gly
CTG GGG CGC GCG GTC GGC AAG GTG GTG ATG GGC ATC GTG GGC GGC
                                                     2700
      760                                            770
val val ser ala val ser gly val ser ser phe met ser asn pro
GTG GTA TCG GCC GTG TCC GGC GTG TCC TCC TTC ATG TCC AAC CCC
                                                          2750
```

```
                           780
phe gly ala leu ala val gly leu leu val leu ala gly leu ala
TTT GGG GCG CTG GCC GTG GGT CTG TTG GTC CTG GCC GGC CTG GCG
                                                              2800
      790                                 800
ala ala phe phe ala phe arg tyr val met arg leu gln ser asn
GCG GCC TTC TTC GCC TTT CGC TAC GTC ATG CGG CTG CAG AGC AAC

                           810
pro met lys ala leu tyr pro leu thr thr lys glu leu lys asn
CCC ATG AAG GCC CTG TAC CCG CTA ACC ACC AAG GAG CTC AAG AAC
      2850
      820                                 830
pro thr asn pro asp ala ser gly glu gly glu glu gly gly asp
CCC ACC AAC CCG GAC GCG TCC GGG GAG GGC GAG GAG GGC GGC GAC
            2900
                           840
phe asp glu ala lys leu ala glu ala arg glu met ile arg tyr
TTT GAC GAG GCC AAG CTA GCC GAG GCC CGG GAG ATG ATA CGG TAC
                  2950
      850                                 860
met ala leu val ser ala met glu arg thr glu his lys ala lys
ATG GCC CTG GTG TCT GCC ATG GAG CGC ACG GAA CAC AAG GCC AAG
                        3000
                           870
lys lys gly thr ser ala leu leu ser ala lys val thr asp met
AAG AAG GGC ACG AGC GCG CTG CTC AGC GCC AAG GTC ACC GAC ATG
                              3050
      880                                 890
val met arg lys arg arg asn thr asn tyr thr gln val pro asn
GTC ATG CGC AAG CGC CGC AAC ACC AAC TAC ACC CAA GTT CCC AAC
                                       3100
                     900          903
lys asp gly asp ala asp glu asp asp leu OP
AAA GAC GGT GAC GCC GAC GAG GAC GAC CTG TGA CGGGGGGTTTGTTGTAAA
                                              3150
TAAAAACCACGGGTGTTAAACCGCATGTGCATCTTTTGGTGTGTTTGTTTGGTACGCCTT
                              3200
TTGTGTGTGTGGGAAGAAAGAAAAGGGAACACATAAACTCCCCCCGGGTGTCCGCGGCCTG
                        3250
TTTCCTCTTTCCTTTGCCCGTGACAAAACGGACCCCCTTGGTCAGTGCCCGATTCCCCCCC
                  3300
ACGCCTTCCTCCACGTCGAAGGCTTTTGCATTGTAAAGCTACCCGCCTACCCGCGCCTCC
 3350                                              3400
CAATAAAAAAAAACAACATACACCAATGGGTCTTATTTGGTATTACCTGGTTTATTTAAA
                           3450
AAGATATACAGTAAGACATCCCATGGTACCAAAGACCGGGGCGAATCAGCGGGCCCCCAT
                     3500
CATCTGAGAGACCAACAAATCGGCCGCGCCGGGCCGTGTCAACGTCCACGTGTCCTGCGCT
                  3550
GCTGCCGTTGACAAGGCCCCGGCCTCCGCGTTGGATGCCTCCGGTTCGGATCC
                  3600
```

14

Primer extension, using a 22bp oligonucleotide (residues 473-494) indicated that the 5'-end of gB mRNA was located at residue 188. The CAT and TATA transcriptional regulatory signals are presumptively at residues 55-62 and 125-131. Starting at the ATG at residues 438-440, there is an open reading frame of 2709 nucleotides which terminates at a TGA stop codon. Two presumptive polyadenylation signals are located in a 3'-non-coding region at residues 3166-3173 and 3409-3416. The protein gB has a high proline content which is consistent with that observed for HSV glycoproteins gC and gD.

The observed amino acid sequence is characteristic of a membrane protein. There is a very hydrophobic region near the carboxy terminus stretching from amino acid residue number 755 to 794, a 40-amino acid sequence which may span the membrane. At the N-terminus the first 56 amino acids are primarily hydrophobic, there being no charged residues from residues 11 to 56. This hydrophobic amino acid domain precedes a region with a high concentration of charged or hydrophilic amino acids. The hydrophobic sequence at the N-terminus may serve as a secretory leader or signal sequence followed by processing signals for cleavage and removal of the secretory leader. The hydrophobic region near the C-terminus can serve as a transmembrane integrating sequence for binding the protein to the cell membrane.

There are eight possible N-linked glycosylation sites as defined by the sequence asn-X-thr/ser (see also Fig. 8). If the first 56 amino acids are removed by processing and each of the potential N-linked glycosylation sites are utilized with the addition of an average 2kd of carbohydrate per site, the molecular weight of the mature protein would be approximately 110kd.

Expression of gB1 in mammalian cells.

Employing the above DNA sequence or fragment thereof, expression was achieved as follows. The vector employed is a mammalian expression vector, referred to as pSV1/dhfr. This 5.63kb plasmid contains 2.8kb of E. coli plasmid pBR328 sequences, including the ampicillin-resistance β-lactamase gene and the origin of replication. The vector also contains a selectable mammalian cell marker, the mouse dihydrofolate reductase cDNA gene (dhfr) (Nunberg et al., Cell (1980) 19:355) linked to the SV40 early promoter, which directs the transcription of dhfr. Additional SV40 sequences, including t antigen splice donor and splice acceptor sites and the polyadenylation sites for early transcripts, are included downstream from the dhfr gene within a 1.65kb BglII-EcoRI fragment.

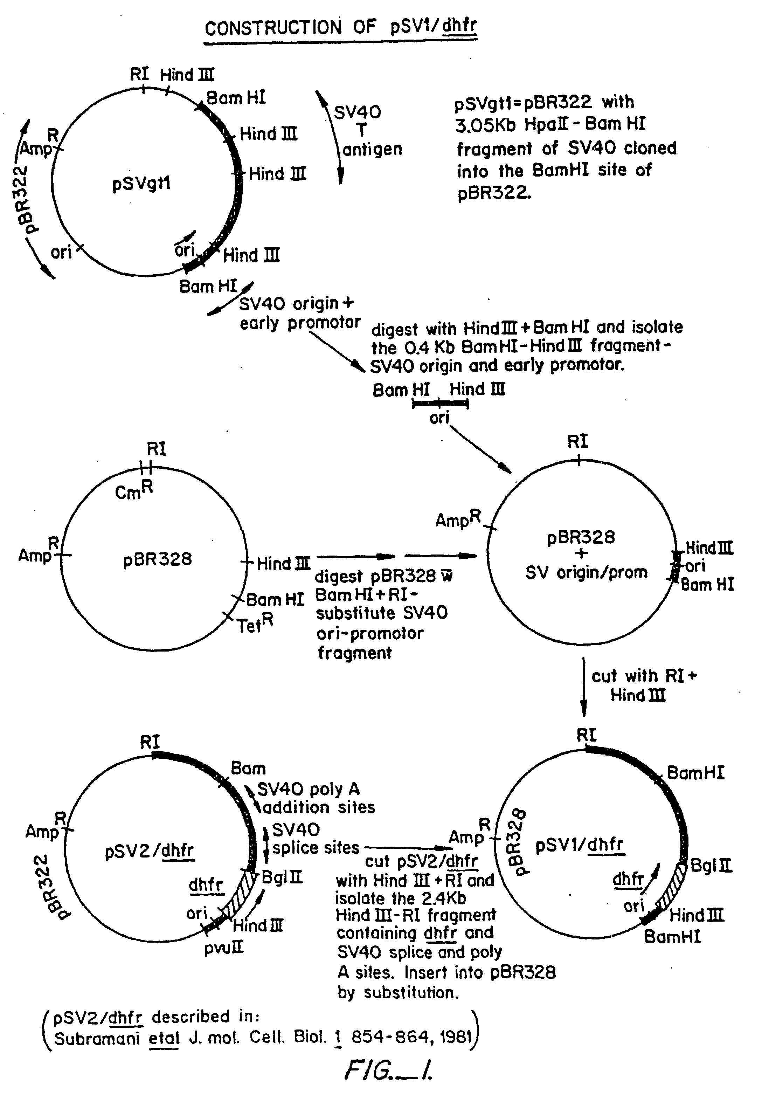

The plasmid pSV1/dhfr was constructed by first isolating the 0.4kb BamHI-HindIII fragment encoding the SV40 origin and early promoter from plasmid pSVgt1. This SV40 fragment was then inserted into plasmid pBR328 by substituting this fragment for the small HindIII-BamHI fragment of pBR328. The dhfr cDNA gene and the SV40 splice sites and poly A sites of pSV1/dhfr were derived from plasmid pSV2/dhfr (Mulligan and Berg, Mol. Cell Biol. (1981) 1: 854-864). The 2.4kb HindIII-RI fragment encoding the dhfr-SV40 sequences was excised from pSV2/dhfr and inserted into the above pBR328 plasmid by substitution for the small HindIII-RI fragment of pBR328. The details of these construction are given in Fig. 1.

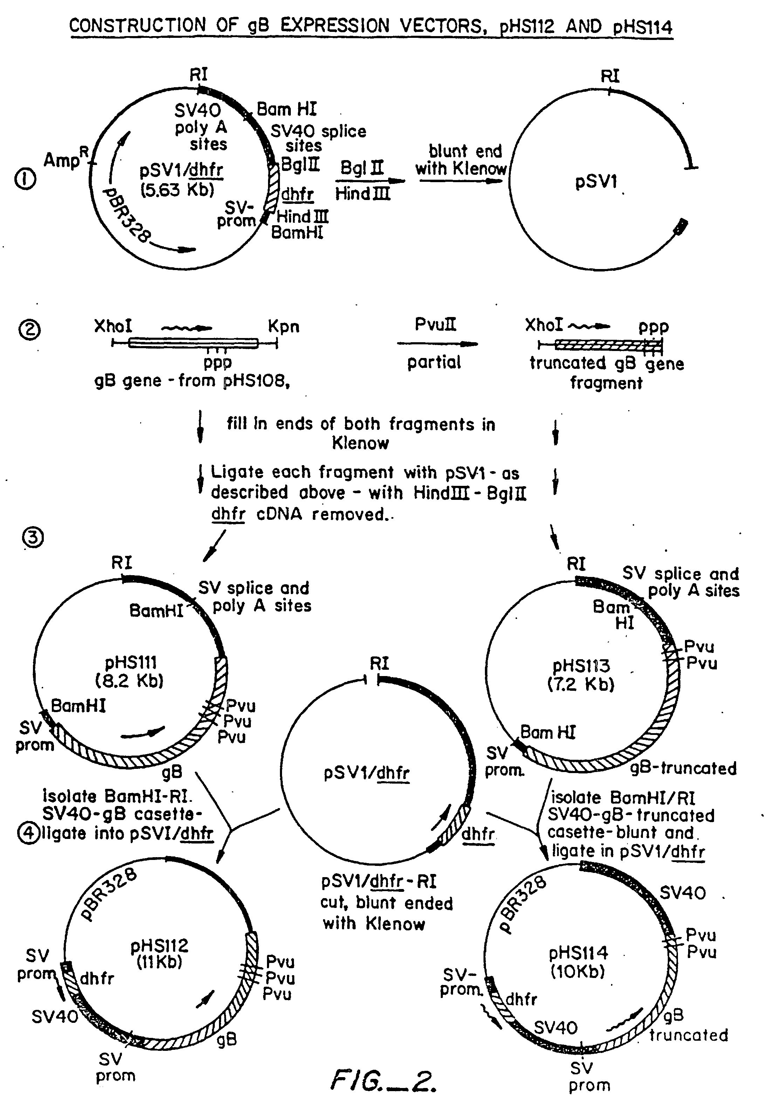

To obtain expression of gB1, two pSV1/dhfr-gB plasmids, pHS112 and pHS114 were constructed. (Fig. 2.) pSV1/dhfr was restricted with BglII and HindIII excising the dhfr cDNA fragment. The resulting fragments are then blunt-ended by filling in the overhangs with the Klenow fragment of DNA polymerase I. A XhoI-KpnI HSV-1 fragment containing the gB gene is isolated from pHS108. A portion is taken and partially digested with PvuII to generate a DNA sequence lacking the 3'-anchor region. The resulting truncated gB1 gene lacks 580bp from the 3'-end of the gene. Both fragments are then blunt-ended with the Klenow fragment of pol I.

Each gB blunt-ended fragment is ligated into the BglII-HindIII restricted pSV1/dhfr vector to provide two sets each of constructs, with the gB1 gene in opposite orientations. The orientations having the XhoI generated terminus proximal to the SV promoter, with the direction of transcription being from the SV promoter to the SV splice sites are selected and designated pHSlll and pHS113 for the complete and truncated gB1 genes, respectively. The two plasmids are then completely digested with EcoRI and partially digested with BamHI to provide a cassette which includes the SV promoter, the gB gene and the SV splice and polyadenylation sites. These fragments are blunt-ended and ligated into EcoRI digested pSV1/dhfr vectors, so as to have the gB1 gene downstream from the dhfr gene and in the same orientation. The complete gB1 gene and truncated gB1 plasmid constructs are designated pHS112 and pHS114, respectively.

The plasmids are then transfected into Chinese hamster ovary (CHO) cells deficient in dhfr using the calcium phosphate precipitation method as described in Materials and Methods. Transfected cells are selected by employing a selective medium lacking thymidine, purines and glycine. Cells were isolated by removal with a Pasteur pipette and propagation in multiwell plates. A number of clones were isolated which were shown to produce gB by immunofluorescence and radioimmunoprecipitation employing a HSV-1 polyclonal antibody or a monoclonal antibody specific for gB. Three cell clones, pHS112-1, pHS112-9 and pHS112-23, were isolated which synthesize an intracellular form of the complete gB protein. The gB made in these cells appears to be glycosylated, since higher molecular weight forms can be detected after a one hour pulse, followed by a 5 hr chase, as compared to non-chased cells and about 10% of the gB is secreted into the media. Five cell clones (pHS114-5, pHS114-6, pHS114-7, pHS114-11 and pHS114-12) expressing the truncated gB were also analyzed and shown to also secrete some gB into the media. One of these cell lines, pHS114-7, was chosen for further amplification with MTX. Clones were initially selected at 0.01, 0.05, 0.10 and 0.3μM MTX. Three clones synthesizing high levels of gB, as detected by immunofluorescence, were isolated from the 0.3μM MTX selections. By radioimmune precipitation, these clones, pHS114-0.3μM-6, 23 and 25, synthesize 2-3 times more gB during a 1hr labeling with $^{35}$S-methionine than the unamplified clone, pHS114-7. Pulse chase experiments indicate that at least 50% of the gB synthesized in these clones during a 1hr pulse is secreted extracellularly by 5hr.

Expression of gB1 in yeast.

Yeast expression was developed as follows: Partial N-terminal amino acid analysis of isolated gB1 was found to have glu, ala, and ala for amino acids 2, 5 and 11, indicating that the N-terminal region of the mature protein was at amino acid 151 of the sequence described previously (after the MboII site).

A cassette was prepared employing the glyceraldehyde-3-phosphate-dehydrogenase promoter region and terminator region. A yeast gene library was prepared by inserting fragments obtained after partial digestion of total yeast DNA with restriction endonuclease Sau3A in lambda-phage Charon 28 (Blattner et al., Science (1977) 196: 161-169). The phage library was screened with DNA complementary to the yeast GAPDH mRNA and the yeast GAPDH gene from one of these clones was subcloned as a 3.5kb BamHI fragment in the BamHI site of pBR322 (pGAP-2). The GAPDH promoting-active fragments were isolated from these clones. A HhaI-HindIII fragment of about 350bp containing the 3' portion of the promoter was obtained by: a) digestion of pGAP-2 with HinfI to generate an approximately 500bp segment which includes the 3' part of the promoter and a region encoding the N-terminal amino acids of GAPDH; b) resection with Bal31 to yield a 400bp fragment lacking the GAPDH coding region (3'-terminus 1 base upstream from the ATG initiator codon); c) addition of HindIII linkers; and d) cleavage with HhaI. A HindIII-HhaI fragment of about 700bp containing the 5' portion of the promoter-was ligated to the 350bp HhaI-HindIII fragment and treated with HindIII. The resulting 1,061bp HindIII fragment was isolated by gel electrophoresis and cloned in pBR322 (pGAP-347). The GAPDH promoter fragment in pGAP347 was isolated by cleavage with BamHI (within the 5' pBR322 flanking region) and partially with HindIII (at the 3' end of the promoter fragment) to provide a 1,407bp fragment containing a 1,061bp region of the GAPDH promoter region and 346bp of.pBR322. This procedure utilized digestion of 50μg of the pGAP-347 with 10 units each of BamHI and HindIII with the resulting fragment purified by preparative gel electrophoresis in 1% agarose.

A synthetic HindIII-XhoII adapter molecule containing the codon for the initiator met and a NcoI site for analysis was synthesized and had the following sequence:

```
AGCTTCCATGGA
    AGGTACCTCTAG.
```

A third fragment was a XhoII-SacII fragment of 1,187bp containing the gB1 coding region.

A fourth fragment containing the GAPDH terminator fragment (approximately 900bp) was isolated by SalI-BamHI digestion of a cloned fragment of the GAPDH gene with its 3' flanking region including the GADPH termination region, so that a portion of the coding region is included with the termination region. The two fragments can be ligated together by means of a SacII-SalI adapter

```
GGACAACTAG
CGCCTGTTGATCAGCT.
```

These five fragments together with the cloning vector were ligated as follows: First, the XhoII-SacII fragment (2 picomoles) was ligated to 100 picomoles of each of the two adapters (HindIII-XhoII, SacII-SalI) using T4 DNA ligase. The product was isolated by preparative gel electrophoresis in 1% agarose, providing a HindIII-SalI fragment. The HindIII-SalI fragment (0.25 picomoles) was ligated in a single step to the 1407bp BamHI-HindIII GAPDH promoter fragment (0.1 picomoles), the 900bp SalI-BamHI terminator (0.1 picomoles) and 0.02 picomoles of BamHI-digested, phosphatased pBR322 in the presence of T4 DNA ligase.

The above reaction product was used to transform E. coli HB101. Plasmids containing the cassette clones in pBR322 were isolated and the correct nucleotide sequence confirmed by DNA sequencing. This plasmid was then digested with BamHI and the BamHI cassette fragment containing the gB1 segment and GAPDH regulatory regions gel isolated and inserted into BamHI digested, phosphatased pC1/1. Plasmid pC1/1 is a derivative of pJDB219 (Beggs, Nature (1978) 275:104) in which the region corresponding to bacterial plasmid pMB9 in pJDB219 is replaced by pBR322 in pC1/1. The pC1/1 plasmid containing the 1187bp gB1 insert and GAPDH promoter and terminator regions was designated pHS127A. This plasmid was then used to transform the yeast strain S. cerevisiae AB103.1 ($\alpha$, pep 4-3, leu 2-3, leu 2-112, ura 3-52, his 4-580). Transformants were initially grown in 1.0ml of leu$^-$ medium and then 50ml of YEPD inoculated with 0.4ml and grown further to an absorbence of 1-3 at 650nm (12hr). The yeast cells were pelleted by centrifugation at 2krpm for 10min at 4°C and resuspended in 50mM Tris-HCl, pH 8, 150mM NaCl, 0.2% Triton X-100, 1mM EDTA and freshly added 1.0mM phenylmethylsulfonyl fluoride and 0.1µg/ml pepstatin. The cells were repelleted and then resuspended in a volume equal to the packed cell volume in the same buffer. An equal volume of acid-washed glass beads (diameter 0.45-0.5mm) was added and the yeast cells disrupted by vortexing at 4°C for 10min total, using 1min intervals.

The tubes were centrifuged for 15min at 14,000xg at 4°C and the supernatant isolated and analyzed on 10% SDS polyacrylamide gel and blotted onto nitrocellulose paper for Western analysis (Burnett, Anal. Biochemistry (1981) 112:195). A polyclonal antibody (DAKO) to HSV-1 was employed as the primary antibody. Expression of an HSV specific protein was observed at about 44kd, the size expected for the gB fragment.

Isolation, cloning and characterization of the gB2 gene.

The gene for glycoprotein gB2 was located by hybridization with specific DNA probes generated from the gB1 coding region. As a first step, a restriction map was determined for the HindIII H fragment of HSV-2 strain 333 which spans map coordinates 0.28-0.39 as shown in Fig. 9.

A random library of HSV-2 strain 333 sequences cloned into the lambda vector L-47.1 (Leonen and Brammar, Gene (1980) 10:249) (obtained from Dr. James Casey, Louisiana State University) was generated by a partial MboI digestion of the viral DNA and cloned into the BamHI site of the vector. The library was screened for plaque hybridization using a KpnI-NdeI fragment of gB1 as a probe. A positive lambda clone was picked and the lambda DNA prepared and digested with SphI (Maniatis et al., Molecular Cloning, Cold Spring Harbor Laboratory, 1982). A Southern blot analysis show that the gB2 information was contained within a 4.2kb fragment. The SphI-digested lambda DNA was ligated to SphI-digested pBR322 and the mixture used to transform E. coli HB101. The appropriate transformant was selected by restriction analysis and confirmed by Southern blot analysis.

Three DNA pieces from the gB1 gene were used as probes to Southern blots of restriction digests of the HindIII H fragment, a 0.93kb PstI-AluI piece from the 5'-end of the gene, a 0.54kb PstI-SalI piece from the central region and a 0.9kb NcoI-NcoI piece from the 3'-end. These fragments have been described previously. The hybridization pattern observed indicated that the gene spanned the two XhoI-XhoI fragments of 2.6kb and 2.75kb. The gB2 gene was cloned in a pBR322 derivative as two overlapping fragments. The 5'-end is contained within pHS203, a 2.6kb XhoI-XhoI clone, and the 3'-end within the adjacent 4.2kb SphI-SphI fragment, pH206, indicated on the above figure.

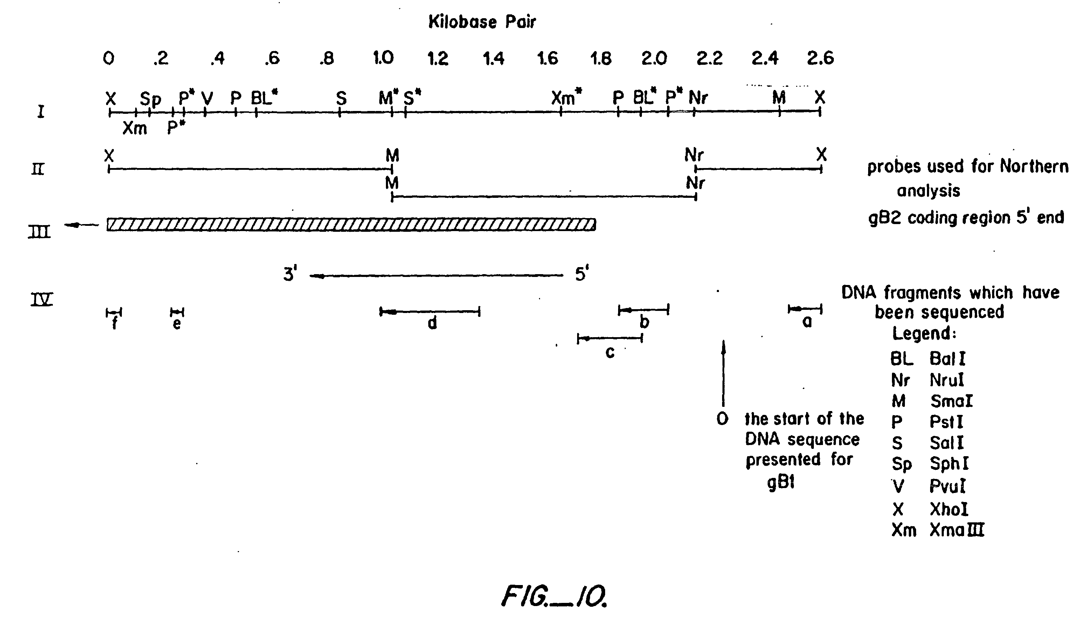

A more detailed restriction map of the pHS203 XhoI subclone is shown in Fig. 10. The figure shows a restriction map of pHS203, a 2.6kb XhoI-XhoI fragment which contains the 5'-end of the gene encoding gB2. Line I shows a restriction map which includes the restriction sites for Bl, BalI.; Nr, NruI; M, SmaI; P, PstI; S, SalI; Sp, SphI; V, PvuI; X, XhoI; Xm, XmaIII. Those restriction sites marked by an asterisk are conserved between the DNA sequences of gB1 and gB2. Line II shows three DNA fragments which were prepared by restriction digestion of pHS203 DNA and isolated by preparative gel electrophoresis and electroelution from a 1% agarose gel. These pieces were used to probe Northern blots of mRNA from HSV-2-infected Vero cells to map precisely the location of the gB2 gene. Line III indicates the direction of transcription from right to left. Line IV locates those DNA fragments of pHS203 which have been sequenced on the genetic map. The start of the DNA sequence, nucleotide 1, for gB1 is also noted by the arrow.

To verify the location of the gB2 gene, the three fragments indicated on Fig. 10 were prepared as probes to Northern blots of poly A$^+$ mRNA isolated from HSV-2 infected Vero cells 12hr after infection. Two of the probes, the 850bp XhoI to SmaI fragment and the adjacent 1150bp SmaI to NruI fragment both hybridized to an abundant mRNA of 3.0kb, the expected size and representation frequency expected for the gB2 message. The third probe, the 470bp NruI-XhoI fragment did not hybridize to the same message.

From a comparison of the HSV-1 and HSV-2 restriction maps, some of the restriction sites are conserved between the two species. To corroborate further the location of the gB2 gene and its homology with the gB1 gene, DNA sequence analysis of selected regions of the gB2 gene were determined.

The sequence of the fragments is shown as follows in Table 3 including a comparison of the sequence of the 5'-end of gB1 and gB2 for other than fragment DNA a.

Table 3

EP 0 628 633 B1

## DNA a

```
                10             20             30             40             50             60
    0   CTCGAGAAGA TGCTGCGGGT CAGCGTCCAC GGCGAGGTGC TGCCCGCCGA CCTCTCGCCG.
                70             80             90            100            110            119
   60   CGGTCGCCAA CGGCTTCGCG GCGCGCGCGC GCTTCTGCGC CCTGACGGCG GGCGCGGGC
```

## DNA b

```
.206 CTGCAGCCGCCCGACCTCCGAAG TCGTTACCGTTACCCGCCCGGCGTATATCTCACGTACGACTCCGACTG
     ***************#***### ** **** ######## ##### ## ###*#### #########*#*#
   1 CTGCAGCCGCCCGACCTCCGAAGGTCTGTACCGCTACCCGCCGGGCGTGTACCTCACGTACAACTCCGACTG

 278 TCCGCTGGTGGCCATCGTCGAGAGCGCCCCCGACGGCTGTATCGGCCCCCGGTCGGTCGTGGTCTACGACCG
     ****************#*****###* ************ ##### ##### *#********### *#*#####
  73 TCCGCTGGTGGCCATCGTCGAGAGCGGCCCCGACGGCTGCATCGGACCCCGCTCGGTCGTGGTTTACGACCG

 350 AGACGTTTTCTCGATCCTCTACTCGGTCCTCCAGCACCTCGCCCCCAGGCTACCTGACGGGGGGGCACGACGG
     ********* ## **************##### #**
 145 AGACGTTTTTTCCATCCTCTACTCGGTCCTGCAG
```
--- -- ----------------------------------------------------------------------

## DNA c

```
 285 TGGCCATCGTCG AGAGCGCCCCCGACGGCTGTATCGGCCCCCGGTCGGTCGTGGTCTACGACCGAGACGTT
     ********#**** ***### ********#####* ***** ***** #*#*#*###*# #*##*###**###*
   1 TGGCCATCGTCGCAGAGCGGCCCCGACGGCTGCATCGGACCCCGCTCGGTCGTGGTTTACGACCGAGACGTT

 357 TTCTCGATCCTCTAC TCGGTCCTCCAGCACCTCGCCCCCAGGCTACCTGACGGGGGGGCACGACGGGCCCCC
     ** ** ********* ######## ***************** #** # * *** *** ** ######
  73 TTTTCCATCCTCTACCTCGGTCCTGCAGCACCTCGCCCCCAGACTAGCGGGCGGCGGGAGGCACCGGCCCCG

 429 GTAGTCCCGCCATGCGCCAGGGCGCCCCCGCGCGGGGGTGCCGGTGGTTCGTCGTATGGGCGCTCTTGGGGT
     *** **********#** ### # * *** * * * *** * **
 145   TAG CCCGCCATGCGCGGGGGGGGGCTTGATTTGGCGCTGGTCGTGGGGGCGCTGGTGGCCGCGGTCGGCG

 501 TGACGCTGGGGGGTCCTGGTGGCGTCGGCGGCTCCGAGTTCCCCCGGCACGCCTGGGGTCGCGGCCGCGACCC
     * ***
 217 TCGCGCCC
```
: ---- ---- ..----------------- ------- ----------------- --------.. ...

**DNA d**

```
 888  AAGGAGAACATCGCCCCGTACAAGTTCAAGGCCACCATGTACTACAAAGACGTCACCGTT TCGCAGGTGTG
      ************************  ***  ******************************* ***  ************
   1  AAGGAGAACATCGCCCCGATCAAATTCAAGGCCACCATGTACTACAAAGACGTGACCTGGGTCGCAGGTGTG

 960  GTTCGGCCACCGCTACTCCCAGTTTATGGGGATCTTTGAGGACCGCGCCCCCGTCCCCTTCGAGGAGGTGAT
      *********************************** ** ***************** **********************
  73  GTTCGGCCACCGCTACTCCCAGTTTATGGGGATATTCCAGGACCGCGCCCCCGTTCCCTTCGAGGAGGTGAT

1032  CGACAAGATC AACGCCAAGGGGGGTCTGTCGGTCCACGGCCAAGTACGTGCGCAACAACCTGGAGACCACCG
      *******  ************** **  *  ****************** **  ******  **************
 145  CGACAAGCATTAACGCCAAGGGGG CTCTGCCTCCACGGCCAAGTACGTCCGGAACAACATGGAGACCACCG

1104  CGTTTCACCGGGACGACCACGAGACCGACATGGAGCTGAAACCGGCCAACGCCCGCGACCCGCACGAGCCGG
      ********************************************* ** *****  ** *  *** ** ************
 217  CGTTTCACCGGGACGACCACGAGACCGACATGGAGCTCAAGCCGGCGAAGGT CGCCACGCGCACGAGCCGG

1176  GGCTGGCACACCACCGACCTCAAGTACAACCCCTCGCGGGTGGAGGCGTTCCACCGGTACGG ACGACGGTA
      ** ***********************************  *****************************  ********  ************
 289  GGGTGGCACACCACCGACCTCAAGTACAACC  TCGCGGGTGGAGGCGTTCCATCGGTACGGCACGACGGTC
```

**DNA e**

```
1950  CTGCAGTTTACGTACAACCACATACAGCGCCATGTCAACGATATGTTGGGCCGCGTTGCCATCGCGTGGTGC
      *************  **************** **  *****  *** ****  *** *  *** ***********
   1  CTGCAGTTTACGTATAACCACATACAGCGCCACGTGAACGACATGCTGGGGCGCATCGCCGTCGCGTGGTGC

2022  GAGCTGCAGAATCACAAGCTGACCCTGTGGAACGAGGCCCGCAAGCTGAACCCCAACGCCATCGCCTCGGCC
      *********
  73  GAGCTGCAG
```

**DNA f**

```
2276  GGGGCAGCTGGGGGGAGAACAACGAGCTGCGGCTGACGCGCGATGCGATCGAGCCGTGCACCGTGGGACACCG
      ************  ******************** **  *****     **
   1  GGGGCAGCTGGGCGAGAACAACGAGCTGCGCCTCACGCGACGCGC
```

A comparison of the homology between the DNA sequences b through f of HSV-2 gB2 and HSV-1 gB1. The top line of each comparison is the gB1 sequence and the number given is the nucleotide base pair of that same

Table 3

sequence. The bottom line is the corresponding gB2 fragment. Loop out regions or spaces have been inserted as required to display the maximum homology.

Expression of gB2 in mammalian cells.

Expression of HSV-2 glycoprotein gB has been achieved in COS cells (transient expression) and in CHO cells (stable cell line secreting gB2) transformed with pHS210 alone or cotransformed with pHS210 and a second plasmid containing dhfr.

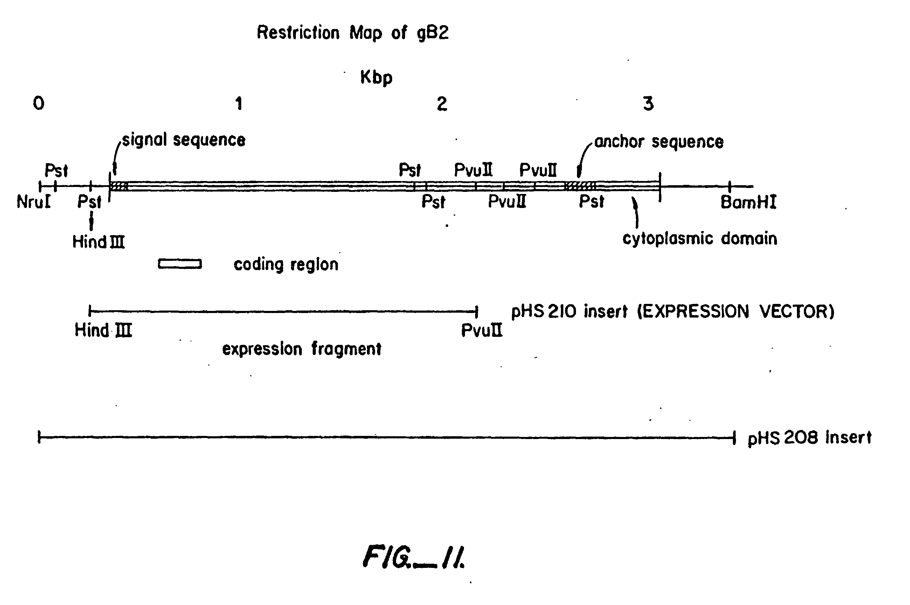

Plasmid pHS210 was constructed as follows. The entire gene was subcloned as a 3.8kb NruI-BamHI fragment in pBR322 to generate pHS208. See Fig. 11. The PstI site at the 5' end of the gene, 100bp to the right (downstream) of the NruI site, was changed to a HindIII site by in vitro mutagenesis in M13. A HindIII to Pvu fragment of 1.9kb was then inserted into pSVI which was obtained by digestion of pSV1/dhfr with HindIII and BglII. See Figs. 1 and 11. For this cloning step pHS208, was cut with PvuII and the end repaired to blunt. The molecule was then cut with HindIII and the 1.9kb HindIII - (PvuII) fragment isolated by gel electrophoresis. Likewise pSV1/dhfr was cut with BglII, repaired to blunt, cut with HindIII and the 4.85kb HindIII-(BglII) vector fragment isolated by gel electrophoresis. These two fragments (1.9kb and 4.85kb) were ligated together to generate pHS210 - the expression plasmid.

Plasmid pHS210 was used directly to transform COS cells. Expression was detected by immunofluorescence using a gB specific monoclonal antibody, F3 A/B, and also using a commercially available polyclonal anti HSV-2 antibody (DAKO) as the primary antibody screen. Plates were coated with the monoclonal antibody. Samples of cell culture medium were added to coated plates, then bound gB2 was detected with the rabbit anti HSV-2 polyclonal antibody (DAKO) followed by horseradish conjugated goat antirabbit IgG.

For CHO cell transformation plasmid pHS210 was used along with a second plasmid containing dhfr as a selective marker in a cotransfection protocol. Expression was detected using an ELISA assay in which ELISA plates were coated with F3 A/B specific monoclonal antibody.

Glycoprotein D

Materials and Methods

All DNA manipulations were done according to standard procedures. See T. Maniatis et al., Molecular Cloning, Cold Spring Harbor Lab., (1982). Enzymes used in cloning were obtained either from New England Biolabs or Bethesda Research Laboratories and employed according to the supplier's directions. Yeast were transformed and grown using a variety of media including selective medium (yeast nitrogen base without leucine); YEPD medium, containing 1% (w/v) yeast extract, 2% (w/v) peptone and 2% (w/v) glucose, and others as appropriate and/or detailed below. In the case of plating medium contained 2% (w/v) agar and for transformation 3% top agar.

Construction of yeast expression vectors containing synthetic DNA fragments coding for polypeptides A and B of the gD gene.

Nucleotide sequences designated gD-A and gD-B based on portions of the amino acid sequence for glycoprotein D of HSV-1 reported by Watson et al., Science (1982) 218:381-384, and employing preferred yeast codons, were devised. The gD-A sequence, which encodes amino acids 253-283 (corresponding to amino acids 258-288, as incorrectly numbered by Watson et al., supra.) of the mature protein, was as follows:

```
                Processing Site
                      ↓
      ValProLeuAspLysArgLeuProProGluLeuSerGluThrProAsnAlaThrGln
       5'-CCTTGGATAAAAGATTGCCACCAGAATTGTCTGAAACCCCAAACGCTACCCAA
         CATGGGAACCTATTTTCTAACGGTGGTCTTAACAGACTTTGGGGTTTGCGATGGGTT

      ProGluLeuAlaProGluAspProGluAspSerAlaLeuLeuGluAspProOP OC
      CCAGAATTGGCTCCAGAAGACCCAGAAGACTCTGCTTTGTTGGAAGACCCATGATAAG-3'
      GGTCTTAACCGAGGTCTTCTGGGTCTTCTGAGACGAAACAACCTTCTGGGTACTATTCAGCT
```

The gD-B sequence, which encodes amino acids 8-23 (corresponding to amino acids 13-28, as incorrectly numbered by Watson et al., supra.) of the mature protein, was as follows:

```
                 Processing Site
                       ↓
ValProLeuAspLysArgSerLeuLysMetAlaAspProAsnArgPheArgGlyLys
 5'-CCTTGGATAAAAGATCTTTGAAGATGGCTGACCCAAACAGATTCAGAGGTAAG
   CATGGGAACCTATTTTCTAGAAACTTCTACCGACTGGGTTTGTCTAAGTCTCCATTC


AspLeuProOP OC
GACTTGCCATGATAAG-3'
CTGAACGGTACTATTCAGCT
```

The sequences each include a KpnI cohesive end at the 5'-end and a SalI cohesive end at the 3'-end. Coding for the mature secreted peptide begins after the LysArg processing site. The 5'-end of each sequence is a modification of the 3'-end of the naturally-occurring α-factor secretory leader and processing signal sequence, where the modification consists of a deletion of three glu-ala pairs and a replacement of a leucine by a proline to create a KpnI site in the nucleotide sequence. The 3'-end of each sequence includes two translational stop codons, OP and OC.

Synthetic DNA fragments having the sequences just described were prepared by synthesizing overlapping ssDNA segments as described by Urdea et al., Proc. Natl. Acad. Sci. USA (1984) 80:7461-7465 using the phosphoramidite method of Beaucage and Caruthers, Tetrahedron Lett. (1981) 27:1859-1862, and annealing and ligating under the following conditions.

The ssDNA fragments were joined as follows: 50 to 100 pmoles of each segment (except the two segments at the 5'-termini) were 5'-phosphorylated with 5.6 units of T4 polynucleotide kinase (New England Nuclear) in 10mM dithiothreitol (DTT), 1mM ATP, 10mM $MgCl_2$, 100ng/ml spermidine, 50mM Tris-HCl, pH 7.8 (total volume: 20µl) for 30 min at 37°C. Additional T4 kinase (5.6 units) was then added and the reaction continued for 30 min at 37°C. The fragments (except for the 5'-termini) were combined, diluted to 40µl with water followed by addition of 60µl of 1M sodium acetate, 12µl of 250mM EDTA, and 5µg of 1mg/ml poly-dA. After heating for 5min at 65°C, the 5' terminal pieces were added, followed by 420µl of ethanol (100%). The solution was chilled for 20min at -80°C, centrifuged, and the pellet was.washed twice with ethanol (100%) and dried. The pellet was redissolved in water (18µl), heated to 100°C for 2 minutes and then cooled slowly over 1.5 hours to 25°C in a water bath.

The annealed fragment pool was ligated in a reaction mixture containing T4 DNA ligase (New England Biolabs, 1200 units) 1mM ATP, 10mM DTT, 10mM $MgCl_2$, 100ng/ml spermidine, and 50mM Tris-HCl, pH 7.8 (30µl). After 1 hour at 14°C, the reaction mixture was partially purified by preparative polyacrylamide gel (7%, native) electrophoresis. The DNA was removed from the appropriate gel slice by electroelution and ethanol coprecipitation with poly dA (5µg).

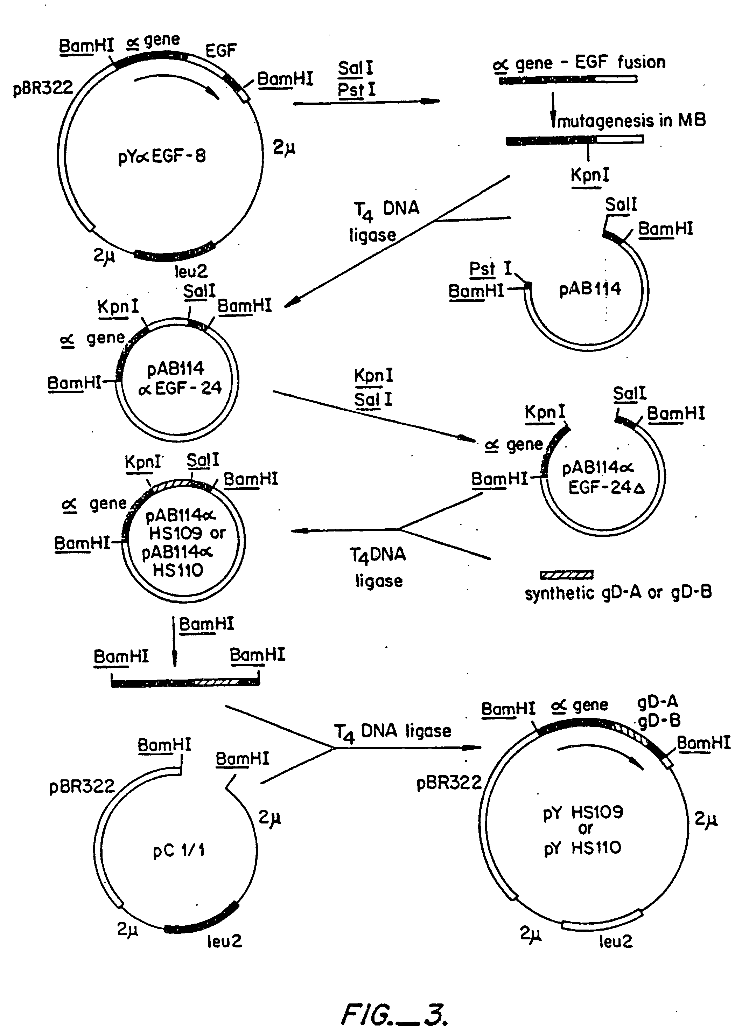

After assembly, the synthetic gD-A and gD-B fragments were substituted into a KpnI/SalI digested bacterial cloning plasmid pAB114αEGF-24, which plasmid was prepared by cloning a mutagenized fragment of pYEGF-8 into pAB114 (see Fig. 3). The plasmids resulting from the insertion were designated pAB114αHS109 (gD-A) and pAB114αHS110 (gD-B).

The preparation of pAB114 was as follows: Plasmid pAB101 was obtained from the screening of a random yeast genomic library cloned in YEp24 (Fasiolo et al., J. Biol. Chem. (1981) 256:2324) using a synthetic 20-mer oligonucleotide probe (5'-TTAGTACATTGGTTGGCCGG-3') homologous to the published α-factor coding region (Kurjan and Herskowitz, Abstracts (1981), Cold Springs Harbor meeting on the Molecular Biology of Yeasts, page 242). Plasmid AB11 was obtained by deleting the HindIII to SalI region of pBR322. An EcoRI fragment of pAB101 carrying the α-factor gene was then inserted into the unique EcoRI site in pAB11 to produce pAB112. Plasmid pAB112 was digested to completion with HindIII, and then religated at low (4µg/ml) DNA concentration to produce plasmid pAB113 in which three 63bp HindIII fragments were deleted from the α-factor structural gene, leaving only a single copy of mature α-factor coding region. A BamHI site was added to plasmid pAB11 by cleavage with EcoRI, filling in of the overhanging ends by the Klenow fragment of DNA polymerase, ligation of BamHI linkers and religation to obtain a plasmid designated pAB12. Plasmid pAB113 was digested with EcoRI, the overhanging ends filled in, and ligated to BamHI linkers. After digestion with BamHI, the resulting 1500bp which carries the single copy of the α-factor gene fragment was gel-purified and ligated to pAB12 which had been digested with BamHI and treated with alkaline phosphatase to produce pAB114, which contains a 1500bp BamHI fragment carrying the α-factor gene.

The preparation of pYEGF-8 was as follows: A synthetic sequence for human epidermal growth factor (EGF) was prepared and ligated to pAB112 (described above) which had been previously completely digested with HindIII and SalI to produce pAB201. The HindIII site lies within the 3'-end of the α-factor gene, and the EGF sequence was inserted using appropriate linkers. The resulting plasmid was designated pAB201.

Plasmid pAB201 (5µg) was digested to completion with EcoRI and the resulting fragments were filled in with DNA polymerase I Klenow fragment and ligated to an excess of BamHI linkers. The resulting 1.75kbp fragment was isolated by preparative gel electrophoresis, and approximately 100ng of this fragment was ligated to long of yeast plasmid pC1/1 (described below) which had been previously digested to completion with restriction enzyme BamHI and treated with alkaline phosphatase. The ligation mixture of the 1.75 kbp fragment carrying the partial α-factor gene fused to the EGF gene and pC1/1 was used to transform E. coli HB101 cells, and transformants were selected based on ampicillin resistance. DNA from one ampicillin resistant clone (designated pYEGF-8) was used to transform yeast AB103 (genotype: MATα, pep 4-3, leu 2-3, leu 2-112, ura 3-52, his 4-580, cir°) cells, and transformants selected based on their leu⁺ phenotype.

Plasmid pC1/1 is a derivative of pJDB219 (Beggs, Nature (1978) 275:104) where the region derived from bacterial plasmid pMB9 has been replaced by pBR322. The pC1/1 plasmid carries genes for both ampicillin resistance and leucine prototrophy.

Plasmid pAB114αEGF-24 was generated by an in vitro mutagenesis procedure which deleted the sequences coding for the glu-ala processing region in the α-factor leader. Plasmid pAB114αEGF-24 was obtained as follows: a PstI-SalI fragment of pYEGF-8 containing the α-factor leader hEGF fusion was cloned in phage M13 and isolated in single-stranded form. A synthetic 36-mer oligonucleotide primer (5'-GGGGTACCTTTGGATAAAAGAAACTCCGACTCCGAA-3') was used as a primer for the synthesis of the second strand using the Klenow fragment of DNA polymerase I. After fill-in and ligation at 14°C for 18 hours, the mixture was treated with $S_1$ nuclease and used to transfect E. coli JM101 cells. Phage containing DNA sequences in which the glu-ala region was removed were located using ³²P-labelled primer as a probe. DNA from positive plaques was isolated, digested with PstI and SalI, and the resulting fragment inserted into pAB114 (described above) which has been previously digested to completion with SalI, partially with PstI and treated with alkaline phosphatase. The resulting plasmid was designated pAB114αEGF-24.

Referring again to Fig. 3, the BamHI-BamHI fragment of pAB114αHS109 or pAB114αHS110 (1588 base pairs for gD-A and 1546 base pairs for gD-B) was excised and ligated into the unique BamHI site of pC1/1. The resulting expression vectors were designated pYHS109 for gD-A and pYHS110 for gD-B.

Expression of gD-A and gD-B polypeptides.

Plasmids pYHS109 and pYHS110 were both used to transform yeast strain AB103.1 (α, pep 4-3, leu 2-3, leu 2-112, ura 3-52, his 4-580, cir°) to leu prototrophy following the procedure of Hinnen et al., Proc. Natl. Acad. Sci. USA (1978) 75:1929-1933. The transformants were grown in 1 L cultures at 30°C in buffered leucine-deficient media to saturation, corresponding to an absorbance of 5 at 650nm. Yeast cell cultures were maintained at saturation for an additional 12 to 24 hrs with shaking at 30°C. The cultures were then harvested, the intact yeast cells pelleted by centrifugation at 3000 RPM, and the resulting supernatant media filtered through a 0.22μ Millipore filter. This fraction was then passed through a C18 reverse phase column, constructed from 8 Seppak units purchased from Waters. The bound material was eluted with 30 ml of 80% (v/v) acetonitrile, 0.1% (v/v) trifluoroacetic acid in water, evaporated to dryness with a Buchii RotoVap and redissolved in 1.6ml of distilled water. This material was separated on a HPLC C18 column monitored at 210nm. The peak corresponding to each respective peptide was collected and its identity confirmed by antigenicity. Each peptide reacted specifically in an ELISA assay using rabbit polyclonal antisera which had been raised against a chemically synthesized gD-B peptide or partial gD-A peptide (residues 256 through 271 of sequence shown in page 9) purchased from Vega Biochemicals. Expression levels, as determined by spectrophotometric measurements of HPLC purified peptides, were on the order of 7.6mg of gD-A per liter of yeast culture ($OD_{650}$=5) and 0.6mg of gD-B per liter of yeast culture ($OD_{650}$=5). These results demonstrate the feasibility of expressing a relatively short portion or fragment of a protein and its secretion from yeast cells using an α-factor expression vector.

Construction of yeast vectors for high level intracellular expression using fragments of the naturally-occurring gD gene.

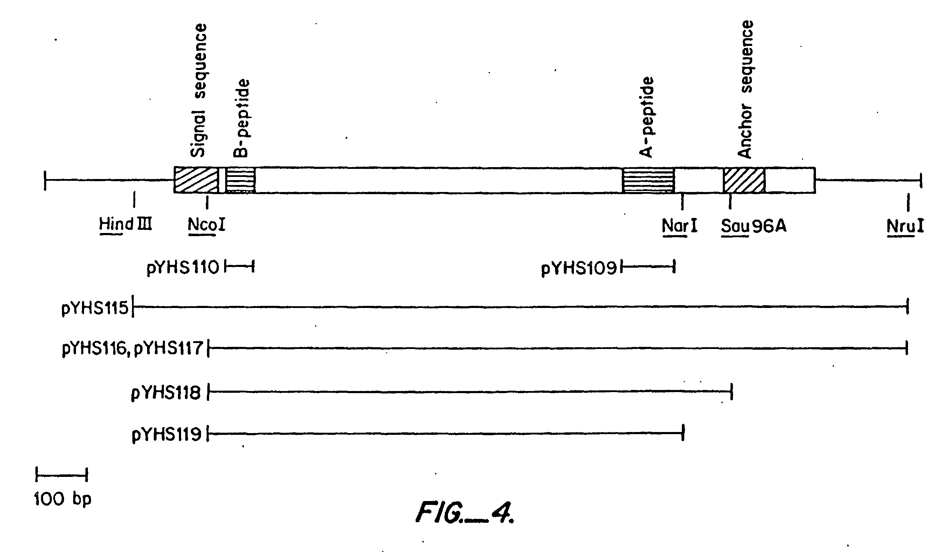

Nucleotide fragments from the naturally-occurring gD gene expressing gD of HSV-1 (gD-1) were also expressed intracellularly in yeast under control of the GAPDH promoter and terminator. A library of EcoRI fragments of HSV-1, strain Patton, cloned into the EcoRI site of pBR322, was made by Dr. Richard Hyman, Hershey Medical Center, Hershey, Pennsylvania. The gD is entirely contained within a 2.9kb SacI fragment within the EcoRI fragment of one clone (clone H) isolated from the HSV-1 library. Clone H was obtained from Dr. Hyman, the 2.9kb fragment was purified by gel electrophoresis and was used for the construction of several expression vectors which differ in the size of the gD fragment cloned and/or the synthetic linkers used in the 5' or 3' ends of the gD fragments. Fig. 4 illustrates the protein coding region (boxed region) and the fragments used for the construction of yeast expression vectors pYHS115, pYHS116, pYHS117, pYHS118 and pYHS119. A description of the construction of each plasmid follows.

Construction of pYHS115.

Plasmid pYHS115 contains the gD gene in a glyceraldehyde-3-phosphate dehydrogenase (GAPDH) expression cassette cloned into the BamHI site of pC1/1 (described hereinabove).

The GAPDH expression cassette was constructed as follows. Three fragments were prepared (as described in detail below):

   (a) A BamHI-HindIII fragment (1407bp) containing 346bp of pBR322 and 1061bp of the GAPDH promoter;
   (b) A HindIII-SalI fragment (1430bp) containing the gD gene, and
   (c) A SalI-BamHI fragment (900bp) containing the GAPDH terminator.

These fragments were ligated together and the mixture was digested with BamHI. The 3.7kb resulting cassette was isolated by gel electrophoresis and ligated to BamHI cut, alkaline phosphatase-treated pC1/1 (Fig. 3).

Fragment (a) was prepared by completely digesting pGAP347 (described below) with BamHI followed by partial digestion with HindIII. The resulting 1407 bp fragment containing 346bp of pBR322 and 1061bp of the GAPDH promoter was isolated by gel electrophoresis.

Construction of pGAP347 was as follows (see copending U.S. application Serial No. 468,589, filed February 22, 1983):

PolyA+ RNA was isolated from S. cerevisiae yeast strain A364A. Double-stranded cDNA was synthesized using AMV reverse transcriptase and E. coli DNA polymerase I. Poly-dC-tails were added to the doublestranded cDNA molecule using deoxynucleotide terminal transferase. Poly-dC-tailed cDNA was annealed to poly-dG-tailed pBR322 and used to transform E. coli HB101. 1000 transformants were screened by colony hybridization to labeled PolyA+ RNA, and a subset further examined by restriction endonuclease mapping, and DNA sequencing. Three clones containing GAPDH sequences were isolated from the pool. One clone (pcGAP-9) contained an insert of about 1200 base pairs and was used for further work.

A yeast gene library was prepared by inserting fragments obtained after partial digestion of total yeast DNA with restriction endonuclease Sau3A in lambda phage Charon 28, according to Blattner, et al., Science (1977) 196: 161-169. Several fragments containing yeast GAPDH coding sequences were isolated by screening the phage library with labeled DNA from pcGAP-9. The yeast GAPDH gene of one of these clones was subcloned in pBR322 as a 2.1kb HindIII fragment (pGAP-1). The GAPDH promoter region was isolated from these clones. A HhaI-HindIII fragment of about 350bp containing the 3' portion of the promoter was obtained by: a) digestion of pGAP-1 with HinfI to generate an approximately 500bp segment which includes the 3' part of the promoter and a region encoding the N-terminal amino acids of GAPDH; b) resection with Bal31 to yield a 400bp fragment lacking the GAPDH coding region (3'-terminus one base upstream from the ATG initiator codon); c) addition of HindIII linkers; and d) cleavage with HhaI. A second HindIII-HhaI fragment of about 700bp containing the 5' portion of the promoter was isolated from pGAP-1, ligated to the 350bp HhaI-HindIII fragment and treated with HindIII. The resulting 1061bp HindIII fragment was isolated by gel electrophoresis and cloned in HindIII digested, alkaline phosphatase treated pBR322 to produce pGAP347.

Fragment (b) was obtained as follows. Clone H, isolated from the HSV-1 Patton library was digested with SacI. A 2.9kb SacI fragment was purified by gel electrophoresis and subsequently digested with HindIII and NruI. The 1430bp HindIII-NruI fragment containing the gD gene (Fig. 3) was purified by gel electrophoresis, ligated to NruI-SalI adaptors of the following sequence:

5'-TGATAAG-3'

ACTATTCAGCT

and digested with SalI.

Fragment (c) was obtained as follows. A 900bp fragment containing the GAPDH terminator was obtained by BamHI and SalI digestion of pUH28 (described under "Construction of pYHS117") and purification by gel electrophoresis.

Construction of pYHS116

pYHS116 contains a gD gene fragment which has a 600bp deletion at the 5' end of the. coding region that comprises most of the signal sequence coding region. To construct pYHS116, two fragments were obtained:

   (a) A BamHI-HindIII fragment (1407bp) containing 346bp of pBR322 and 1061bp of the GAPDH promoter. This fragment was obtained as described under "Construction of pYHS115."

(b) A NcoI-BamHI fragment (2150bp) containing the partial gD gene followed by the GAPDH terminator. This fragment was obtained by BamHI/NcoI digestion of pYHS115 (described previously) and purification by gel electrophoresis. A HindIII-NcoI chemically synthesized adaptor of the following sequence

```
                  metval
        5'-AGCTTAACATGGTC-3'
                  ATTGTACCAGGTAC
            ↑                        ↑
        HindIII              NcoI
```

was ligated to the fragment. This adaptor provides for the first two codons (met and val) fused in the correct reading frame to the partial gD.

Fragments (a) and (b) were ligated together and subsequently digested with BamHI. The resulting 3.5kb cassette was isolated by gel electrophoresis and ligated to BamHI cut, alkaline phosphatase treated pC1/1.

Construction of pYHS117

Plasmid pYHS117 contains the same partial gD gene clone in pYHS116, fused in reading frame to 7 extra codons at the 5'-end, which code for the first 7 amino acids of the GAPDH structural gene. To construct pYHS117 two fragments were obtained.

(a) An NcoI-SalI digested vector (6.8kb) comprising pBR322 sequences, the GAPDH promoter fused to the first 7 codons of the structural gene and the GAPDH terminator. This vector was prepared by NcoI digestion of pUH28 (described below), followed by a partial digestion with SalI and purification by gel electrophoresis.

(b) An NcoI-SalI fragment (1430bp) containing a partial gD gene. This fragment was obtained by NcoI-SalI digestion of pYHS115 (described previously in Section II.1.) and purification by gel electrophoresis.

These two fragments were ligated together to yield a pBR322 derived vector which contains a partial gD gene fused in reading frame to the 7 first codons of GAPDH gene, flanked by the GAPDH promoter in its 5' end and by the GAPDH terminator in its 3' end. The gD expression cassette was obtained by digesting this plasmid with BamHI and purifying a 3.4kb fragment by gel electrophoresis. This fragment was ligated to BamHI digested, alkaline phosphatase treated pC1/1 to produce pYHS117.

Plasmid pUH28 contains the coding and 3' non-coding regions of the hepatitis B surface antigen (HBsAg) gene fused in incorrect reading frame to the first 7 codons of the GAPDH structural gene. This fusion is flanked in its 5' end by the GAPDH promoter and in its 3' end by part of the GAPDH coding region followed by the GAPDH terminator. This plasmid was constructed so as to have an NcoI site at the 3' end of the first 7 codons of the GAPDH gene with the following sequence:

```
                  met
        5'-AAACAAAATGGTTAGAGTTGCTAATTCC-3'
           TTTGTTTACCAATCTCAACGATTAAGGGTAC
        3'GAPDH        5'GAPDH            NcoI site
        promoter    coding region
```

When this NcoI end is ligated to the partial gD fragment (b, described above) the correct reading frame for the gD protein is regenerated. The SalI site used in the preparation of fragment a (described above) is at the 5' region of the GAPDH terminator. Therefore, a deletion of the sAg coding plus non-coding regions and GAPDH coding region was obtained by digesting pUH28 with NcoI and partially with SalI.

The construction of pUH28 involves cloning of a fragment that contains the HBsAg coding and 607bp of 3' non-coding region prepared from pHBS5-3 Hae2-1 (described below) into the GAPDH containing vector pGAP$_2$' (described below). To prepare the fragment, pHBS5-3 Hae2-1 was linearized by PstI digestion, partially digested with NcoI and a PstI-NcoI fragment of 1.9kb containing pBR322 sequences, HBsAg coding and 3' sequences was purified by gel electrophoresis. This fragment was subsequently digested with EcoRI and a 1.2kb NcoI-EcoRI fragment containing the HBsAg coding and 3' non-coding regions was purified by gel electrophoresis. Plasmid pGAP$_2$' was linearized with XbaI and treated with Bal31 to remove approximately 100bp. The plasmid was subsequently digested with NcoI and a vector fragment of about 9kb was purified by gel electrophoresis. The NcoI ends of the vector and the 1.2kb NcoI-EcoRI fragment encoding HBsAg were ligated. The recessed end was filled in with Klenow and the resulting blunt end was ligated to the blunt end of the vector obtained by Bal31 digestion to produce pUH28.

pHBS5-3 Hae2-1 is a plasmid that contains the HBsAg coding region and 607bp of 3' flanking sequences. This plasmid is a derivative of pHBS5-3 which contains the same insert but only 128bp of 3' untranslated region instead of 607bp. Plasmid pHBS5-3 has been previously described in copending U.S. application, serial no. 609,540, filed May 11, 1984, (pp. 13-14). pHBS5-3 Hae2-1 was constructed as follows. The HBV genome (3.2kb) was excised from pHB-3200 (Valenzuela et al., Nature (1979) 280:815-819) by restriction digestion with EcoRI. The 3.2kb fragment was purified by gel electrophoresis and was recircularized by ligation of the EcoRI sticky ends. This closed HBV genome was digested with HaeII, which cuts in the 3' non-coding region. Recessed ends were filled in with Klenow and HindIII linkers were ligated. The DNA was cut with HindIII and subsequently with XbaI, which has a single site in the HBS coding region. A 1.2kb XbaI-HindIII fragment containing 586 base pairs of the coding sequence of HBV and 607 base pairs of the 3' non-coding region was isolated by gel electrophoresis. This fragment was cloned into pHBS5-3 previously cut with XbaI and HindIII and treated with alkaline phosphatase, to yield pHBS5-3 Hae2-1.

pGAP-2 is a pBR322 derived vector which contains a BamHI insert that has the GAPDH coding sequence, 5' and 3' flanking regions. There are two XbaI sites in this plasmid: one in the coding region and one in the 3' flanking sequences. pGAP-2' is a derivative of pGAP-2 in which the XbaI site present in the 3' flanking region has been eliminated. For this purpose, 50µg of pGAP-2 were partially digested with XbaI, treated with Bal31 to remove 25 base pairs per end, and ligated. The plasmids were used to transform E. coli HB101 and the transformants were selected for loss of the XbaI site in the 3' flanking region.

Construction of pYHS118

This vector contains a partial gD gene with deletions in two regions: a 600bp deletion in the 5'-end coding region which comprises most of the signal sequence coding region and a 1300bp deletion in the 3'-end coding region which includes most of the anchor sequence coding region. It also contains 7 extra codons from the GAPDH gene coding region fused in reading frame at the 5' end of the gD gene, similar to pYHS117. Plasmid pYHS118 was constructed as follows. pYHS115 was digested with NcoI and SalI, the resulting 1430bp fragment containing the partial gD was purified by gel electrophoresis and submitted to digestion with NarI (Fig. 4). The two resulting fragments (fragment a: 873bp containing 5' end and fragment b: 411bp containing 3' end) were independently isolated by gel electrophoresis. Fragment b was subsequently digested with Sau96A to yield three fragments which were separated by gel electrophoresis. The 87bp Nar-Sau96A fragment was recovered from the gel and was ligated to Sau96I-SalI synthetic adaptors of the following sequence:

```
            gly leu ile ala STOP
        5'-GGC CTG ATC GCG TAG-3'
              G ACT AGC GCATCAGCT
              ↑                    ↑
            Sau96A               SalI
```

The NarI-(Sau96A)SalI fragment (102bp) was digested with SalI, purified by gel electrophoresis and ligated with fragment a (previously described). The resulting NcoI-SalI fragment (975bp) was ligated to NcoI-SalI digested and gel purified pUH28 as described under "Construction of pYHS117." The resulting pBR322 derived vector was digested with BamHI and a 3.1kb fragment containing the gD expression cassette was purified by gel electrophoresis. This cassette was ligated to BamHI digested, alkaline phosphatase treated pC1/1 to produce pYHS118.

Construction of pYHS119

This vector contains a partial gD gene with deletions in two regions: a 600bp deletion in the 5' end coding region which comprises most of the signal sequence coding region and a 2400bp deletion in the 3' end coding region which includes all the anchor sequence coding region and about 700bp upstream of the anchor sequence. It also contains 7 extra codons from the GAPDH gene coding region fused in reading frame at the 5' end of the gD gene, as pYHS117 and pYHS118. Plasmid pYHS119 was constructed as follows. pYHS115 was digested with NcoI and SalI, the resulting 1430bp fragment containing the partial gD was purified by gel electrophoresis and subsequently digested with NarI. The 873bp NcoI-NarI fragment was isolated by gel electrophoresis. A synthetic adaptor of the following sequence:

$$5'\text{-CGCCGCAAATCTAC-3'}$$
$$\text{GGCGTTTAGATCAGCT}$$

$$\uparrow \qquad\qquad \uparrow$$
$$\underline{\text{NarI}} \qquad\qquad \underline{\text{SalI}}$$

which provides complementary nucleotides to the NarI 5' overhang, 3 codons in reading frame, a stop codon and a 5' overhang of SalI, was ligated to the 873bp NcoI-NarI fragment then digested with SalI. The resulting NcoI-SalI fragment was ligated to pUH28 which had been previously completely digested with NcoI and partially digested with SalI and purified by gel electrophoresis as described under "Construction of pYHS117." The resulting pBR322 derived vector was digested with BamHI and a 2.2kb fragment containing the gD expression cassette was purified by gel electrophoresis. This cassette was ligated to BamHI digested, alkaline phosphatase treated pC1/1 to produce pYHS119.

Synthesis of gD from vectors containing partial or complete gD gene.

Plasmids pYHS115, 116, 117, 118 and 119 were used to transform yeast strain AB103.1 ($\alpha$, pep 4-3, leu 2-3, leu 2-113, ura 3-52, his 4-580, cir°) following the procedure of Hinnen et al., supra. The transformants were grown to an $OD_{650}$=3 at 30° in YEPD media. The cultures were then harvested by pelleting the yeast cells at 3000 RPM. Cells were spheroplasted with zymolyase and subsequently osmotically lysed in a hypotonic solution. Membranes were spun down in an eppendorf centrifuge, and the pellet was solubilized in 0.1% SDS with protease inhibitors for 16 hours at 4°C. The suspension was centrifuged and total protein, as well as gD specific protein, was determined in both soluble and insoluble fractions. Expression of the gD gene in each of the above described constructions was detected by Western Blot hybridization (Towbin et al., Proc. Natl. Acad. Sci. USA (1979) 76:4350). For this purpose protein samples were submitted to SDS-polyacrylamide gel electrophoresis (Laemmli, Nature (1970) 227:680) and electroblotted onto nitrocellulose filters (Towbin, et al., supra.). The filter was preincubated with goat serum and subsequently treated with a rabbit polyclonal antibody raised against HSV-1 (Dako). The filter was then incubated with a second goat anti-rabbit antibody conjugated with horseradish peroxidase (Boehringer-Mannheim) and finally it was incubated with horseradish peroxide color development reagent (Bio-Rad) and washed. The results indicate that immunoreactive material is being synthesized in yeast AB103.1 strain transformed with the gD expression vectors, with the exception of transformants containing pYHS115. In all other cases, gD protein corresponds to 0.1 to 0.5% of total yeast cell protein.

Vaccine Trials

Mice Experiments

Experiment 1

Outbred female Swiss Webster mice (Simonsen) were immunized on day 1 with a 1.1 mixture of recombinant vaccine preparation and complete Freund's adjuvant. One half the dose was administered subcutaneously, the other half intraperitoneally. The mice received either 20μg recombinant gD protein or 5μg recombinant gB protein. Mice were boosted on day 20 with the same dose and vaccine preparations, but incomplete Freund's adjuvant was used to make the emulsion. They were bled by cardiac puncture on day 28. Total lectin affinity purified Patton strain HSV glycoproteins served as a positive vaccine control, and a recombinant Feline leukemia envelope B protein was used as a negative control. A second boost was given on day 41 and animals were bled again on day 53. Sera from the mouse bleeds were collected and assayed for antibody levels by ELISA and plaque reduction neutralization assay. The results of this experiment are summarized in Table 4 below.

| Animal | Immunogen | Dose | ELISA Titer[a] | Neutralization Titer[b] |
|---|---|---|---|---|
| | | | **BLEED #1** | |
| 1 | recombinant gD | 20 µg | 1:284 | < 1:20 |
| 2 | recombinant gD | 20 µg | 1:77 | < 1:20 |
| 3 | recombinant gD | 20 µg | 1:142 | < 1:20 |
| 4 | recombinant gB | 5 µg | 1:41750 | |
| 5 | recombinant gB | 5 µg | 1:24547 | 1:640 |
| 6 | env B | 20 µg | < 1:10 | < 1:20 |
| 7 | total herpes glycoproteins | 20 µg | 1:175 | 1:125 |
| | | | **BLEED #2** | |
| 1 | recombinant gD | 20 µg | 1:250 | |
| 2 | recombinant gD | 20 µg | 1:50 | |
| 3 | recombinant gD | 20 µg | 1:81 | |
| 4 | recombinant gB | 5 µg | 1:17507 | |
| 5 | recombinant gB | 5 µg | 1:24155 | |
| 6 | env B | 20 µg | < 1:10 | |
| 7 | total HSV glycoproteins | 20 µg | 1:1468 | |

[a] ELISA with 20 µg/ml total HSV-1 (Patton) glycoproteins as antigen, 50% endpoint.

[b] Plaque reduction neutralization assay with complement, HSV-1 (McIntyre), 50% endpoint.

EP 0 628 633 B1

Experiment 2

The in vivo response of mice to the vaccine candidates was also examined by challenging immunized animals with a lethal dose of virus. Female Swiss Webster mice were immunized with the vaccine candidates. The first injections were done using 20μg recombinant gD or 5μg recombinant gB mixed in an equal volume of complete Freund's adjuvant. One half the dose was given intraperitoneally, the other half subcutaneously on day 1. Mice were boosted 2 and 4 weeks later in the same manner with incomplete Freund's adjuvant in the emulsion. Control animals received 20μg of lectin purified total HSV glycoproteins previously described or 23μg of a control yeast plus CHO cell extract. Mice were bled one week after the final boost by cardiac puncture. Sera were collected and assayed for antibody titers by ELISA. Animals were challenged fifteen days later with 3.25 x $10^8$ pfu of HSV-1 (Patton) (2 $LD_{50's}$) given intraperitoneally. They were monitored for a 14 day time period for the appearance of clinical signs or morbidity. Mice surviving the virus challenge were observed for ten additional days at which time they were bled and the sera examined by ELISA. The results of this experiment are summarized in Table 5 below.

## TABLE 5:  Experiment 2 Protection of Mice Against Virus Challenge

| Group | Animal | Immunogen | Dose | ELISA Titer † | % Survival †† | ELISA Titer * |
|-------|--------|-----------|------|---------------|---------------|---------------|
| 1 | A | recombinant gD | 20µg | 1:98 | 40 (2/5) | 1:862 |
|  | B |  |  | 1:146 |  |  |
|  | C |  |  | n.d ** |  | 1:1514 |
|  | D |  |  | 1:355 |  |  |
|  | E |  |  | 1:106 |  |  |
| 2 | A | recombinant gB | 5µg | 1:1928 | 80 (4/5) |  |
|  | B |  |  | 1:1097 |  | 1:2660 |
|  | C |  |  | 1:2660 |  | 1:1539 |
|  | D |  |  | 1:1641 |  | 1:9641 |
|  | E |  |  | 1:13737 |  | 1:7573 |
| 3 | A | total HSV glycoproteins | 20µg | 1:2339 | 100 (4/4) | 1:1928 |
|  | B |  |  | 1:172 |  | 1:965 |
|  | C |  |  | 1:2660 |  | 1:5488 |
|  | D |  |  | 1:2883 |  | 1:3026 |
| 4 | A | control cell extracts | 23µg | <1:25 | 20 (1/5) | 1:734 |
|  | B |  |  | 1:85 |  |  |
|  | C |  |  | 1:51 |  |  |
|  | D |  |  | <1:25 |  |  |
|  | E |  |  | <1:25 |  |  |

† ELISA with 10µg/ml total HSV-1 (Patton) glycoproteins as antigen, 50% endpoint.

†† Percent of mice surviving 14 days after challenge.  (Number of surviving mice over total number.)

** Not done.  No blood was obtained from this mouse for assay.

* Sera tested from mice surviving virus challenge.

EP 0 628 633 B1

Guinea Pig Experiments

Experiment 1

Eighty-five weanling Hartley guinea pigs were divided into 10 groups as described in Table 6 below. The initial immunization contained 10-100µg of glycoprotein in complete Freund's adjuvant in a volume of 100-200µl injected into the hindlimb footpads, followed four weeks later by the same vaccine given intramuscularly to each hindlimb. Four weeks after the second immunization the animals were intravaginally inoculated with the HSV-2 MS strain. The animals were evaluated daily for 14 days to determine the severity of primary infection. The severity of the primary infection was determined by clinical observation of the lesions. The lesions were scored according to the following manifestation:

0   No clinical manifestations of infection
1   Redness, swelling
2   Few small vesicles
3   Several large vesicles
4   Several large ulcers, maceration

The results of this experiment are summarized in Table 6 below. The scores represent group averages.

According to the present invention, novel vaccines are provided effective against Herpes Simplex Virus Types

TABLE 6

SEVERITY OF PRIMARY INFECTION IN GUINEA PIGS CHALLENGED WITH HSV-2

| Group # | Immunogen | Score[a] | Range | Number of Animals |
|---|---|---|---|---|
| 1 | Control, yeast plus CHO cell extracts plus CFA | 3.7 ± 0.1 | 3-4 | 10 |
| 2 | 10 μg recombinant gD plus CFA | 1.3 ± 0.3 | 0-3 | 8 |
| 3 | 100 μg recombinant gD plus CFA | 0.2 ± 0.2 | 0-1 | 7 |
| 4 | 20 μg recombinant gB plus CFA | 0.2 ± 0.1 | 0-2 | 6 |
| 5 | 20 μg recombinant gB plus 10 μg recombinant gD plus CFA | 0.7 ± 0.2 | 0-2 | 8 |
| 6 | 50 μg Total HSV-1 glycoproteins plus CFA | 0.0 | N/A | 7 |
| 7 | 50 μg Total HSV-1 glycoproteins | 0.4 ± 0.1 | 0-1 | 7 |
| 8 | 50 μg Total HSV-2 glycoproteins plus CFA | 0.0 | N/A | 8 |
| 9 | 50 μg Total HSV-2 glycoproteins | 0.4 ± 0.2 | 0-1.5 | 8 |
| 10 | Control, no treatment | 3 ± 0.3 | 0-4 | 16 |

a  Seven days post challenge

EP 0 628 633 B1

1 and 2. These vaccines employ recombinant gB and gD or related polypeptides either separately or together.

Although the foregoing invention has been described in some detail by way of illustration and example for purposes of clarity of understanding, it will be obvious that certain changes and modifications may be practiced within the scope of the appended claims.

The following E. coli HB101 strains were deposited at the A.T.C.C., where the plasmid indicates the plasmid employed to transform the strain: pHS203; pHS112; pHS114; pHS127A and pHS206 were deposited on April 4, 1984, and assigned Accession Nos. 39649-39653, respectively; pYHS109 and pYHS118 were deposited on July 11, 1984, and given Accession Nos. 39762 and 39763 respectively.

## Claims

1. A vaccine comprising an effective amount to produce an immune response in a mammalian host of at least one of HSV glycoprotein D or an immunogenically active fragment thereof, prepared by expression of DNA constructs in yeast.

2. The vaccine of Claim 1, wherein said fragments are about 1500 to 5000 daltons molecular weight.

3. A method for preparing vaccines against Herpes Simplex Virus, comprising
   preparing at least one of HSV glycoprotein D or am immunogenically active fragment of HSV glycoprotein D by expressing DNA constructs in yeast.

4. The method according to Claim 3, wherein said fragments are about 1500 to 5000 daltons molecular weight.

## Patentansprüche

1. Impfstoff, umfassend eine zur Erzeugung einer Immunantwort in einem Säuger-Wirt wirksame Menge von mindestens dem HSV-Glycoprotein D oder einem immunogen aktiven Fragment davon, hergestellt durch Expression von DNA-Konstrukten in Hefe.

2. Impfstoff nach Anspruch 1, wobei die Fragmente ein Molekulargewicht von etwa 1500 bis 5000 Dalton haben.

3. Verfahren zur Herstellung von Impfstoffen gegen Herpes Simplex-Virus, umfassend das Herstellen von mindestens dem HSV-Glycoprotein D oder einem immunogen aktiven Fragment vom HSV-Glycoprotein D durch Expression von DNA-Konstrukten in Hefe.

4. Verfahren nach Anspruch 3, wobei die Fragmente ein Molekulargewicht von etwa 1500 bis 5000 Dalton haben.

## Revendications

1. Vaccin comprenant une quantité efficace pour produire une réponse immune dans un mammifère hôte d'au moins une glycoprotéine D HSV ou un fragment immunogéniquement actif de celle-ci, préparé par l'expression de constructions d'ADN dans une levure.

2. Vaccin selon la revendication 1, dans lequel lesdits fragments sont d'environ 1500 à 5000 daltons de poids moléculaire.

3. Procédé pour préparer des vaccins contre le Virus Simplex de l'Herpès comprenant la préparation d'au moins une glycoprotéine D de HSV ou un fragment immunogéniquement actif de la glycoprotéine D de HSV, par expression des constructions d'ADN dans la levure.

4. Procédé selon la revendication 3, dans lequel lesdits fragments sont d'environ 1500 à 5000 daltons de poids moléculaire.

## CONSTRUCTION OF pSV1/dhfr

pSVgt1 = pBR322 with 3.05Kb HpaII - BamHI fragment of SV40 cloned into the BamHI site of pBR322.

digest with HindIII + BamHI and isolate the 0.4 Kb BamHI - HindIII fragment - SV40 origin and early promotor.

digest pBR328 w̅ BamHI + RI - substitute SV40 ori - promotor fragment

cut with RI + HindIII

cut pSV2/dhfr with HindIII + RI and isolate the 2.4Kb HindIII - RI fragment containing dhfr and SV40 splice and poly A sites. Insert into pBR328 by substitution.

( pSV2/dhfr described in: Subramani etal J. mol. Cell. Biol. 1 854-864, 1981 )

FIG.—1.

CONSTRUCTION OF gB EXPRESSION VECTORS, pHS112 AND pHS114

FIG._2.

FIG._3.

Anchor sequence

Sau96A

NarI

A-peptide

pYHS109

pYHS110

pYHS115

pYHS116, pYHS117

pYHS118

pYHS119

B-peptide

Signal sequence

NcoI

HindIII

NruI

*FIG.\_4.*

100 bp

FIG._5.

FIG.—6.

FIG.—7.

EP 0 628 633 B1

Kilobase Pairs

0    0.5    1.0    1.5    2.0    2.5    3.0    3.5    4.0

3'                                              5'

(A)

B  K    Bs Nc Sc P  Xm  Pv   Pv  Pv    P P  BL Nc    S         Xm   BL P X        X

Nc                                                                    Sc

3'                              central              5'

Nc        Nc              P              S    A              P         probes for mapping of gB2

FIG._8.

HSV-2  333    Hind III  H Map

Kilobase Pairs

0    1    2    3    4    5    6    7    8    9    10   11   12   13   14   15   16   17

E
E              E                        Hp                                    E   Bs

H  B X    Bs    K  B  Bg   K S   Hp    X    B Bs B  Bs X S        X    K  Bs   Bg    X    H B S
                                             *  *            *                            Legend:

pHS 203                                                                          B    Bam HI
                                                                                Bg   Bgl II
pHS 206                                                                          Bs   Bst E II
                                                                                H    Hind III
                                                                                Hp   Hpa I
gB2 coding region                                                               E    EcoRI
                                                                                K    KpnI
3' ◄———— 5'                                                                      S    Sph I
                                                                                X    Xho I
FIG._9.                                                                          ⋀⋀⋀ pBR322

FIG._10.

## Restriction Map of gB2

**Kbp**

FIG.— 11.

EP 0 628 633 B1